# EUROPEAN PATENT APPLICATION

(11) **EP 4 223 281 A1**
(43) Date of publication of application: **09.08.2023**
(21) Application number: 22175496.3
(22) Date of filing: 25.05.2022
(51) Int. Cl.: A61K 9/00, A61K 9/51, A61K 47/58, A61K 47/62, A61K 47/69, A61P 35/00

(54) **LIGHT-CONTROLLABLE SELF-ASSEMBLED COMPLEX AND NANOSYSTEM INCLUDING THE SAME**

(30) Priority: 03.02.2022 KR 20220014028
(71) Applicant: Korea University Research and Business Foundation, Seoul 02841 (KR)
(72) Inventor: Kang, Heemin, 06344 Seoul (KR); Kim, Yuri, 04073 Seoul (KR); Bae, Gun-Hyu, 18477 Gyeonggi-do (KR); Lee, Sung-Gue, 15002 Gyeonggi-do (KR); Kang, Na-Yeon, 02857 Seoul (KR)
(74) Representative: Meissner Bolte Partnerschaft mbB

(57) **Abstract**

Discloses is a technology of collecting a biological sample, delivering a substance into a living body, and regulating macrophage adhesion and polarization, by controlling a self-assembled complex and a nanosystem including the same by irradiation with light.

## Description

### BACKGROUND

### 1. Technical Field

The present invention relates to a light-controllable self-assembled complex and a nanosystem including the same, and more particularly, to a light-controllable self-assembled complex, which is isomerized by absorption of light, and a nanosystem including the same.

### 2. Related Art

Light offers unique opportunities to address molecular systems non-invasively, in a highly selective manner, and with high space and time resolution. Compounds whose shape, biological and chemical activity, dielectric constant and refractive index can be switched reversibly under the control of light can find extensive applications in areas that range from molecular probes to molecular nanotechnology and photopharmacology.

Azobenzene and its derivatives are one of the primary examples of such compounds. Their favorable properties such as photostability and functional activity under a wide range of experimental conditions have paved the way for the further development and application of photochromic systems in many aspects. The basis of the functional behavior of azobenzene is the reversible *trans* (E) → cis (Z) isomerization of the N=N bond upon photoexcitation. Therefore, the absorption spectrum of azobenzene has long attracted attention.

In addition, there is a need for testing and treatment methods that are harmless to the living body and do not damage the living body as much as possible, when applied to the living body.

There is a need to develop a technology that can be applied non-invasively to a living body by using the photoswitching properties of azobenzene and its derivatives.

### [Related Prior Art Documents]

### [Patent Documents]

Korean Patent Application Publication No. 10-2014-0107810 (published on September 5, 2014)

### SUMMARY

An object of the present invention is to provide a technology of collecting a biological sample, delivering a substance into a living body, and regulating macrophage adhesion and polarization, by controlling a self-assembled complex and a nanosystem including the same by irradiation with light.

According to one aspect of the present invention, embodiments of the present invention may include a light-controllable self-assembled complex including: a polymer complex including a ligand and a negatively charged polymer; and a positively charged photoswitchable isomer bound to the polymer complex, wherein the photoswitchable isomer is present as a cis- or trans-isomer and undergoes reversible cis-trans isomerization in response to light, and the volume of the light-controllable self-assembled complex reversibly swells or shrinks due to the isomerization of the photoswitchable isomer.

In one embodiment, the self-assembled complex may reversibly swell and deswell in response to light in an environment with a aqueous liquid, the self-assembled complex may be present in the form of a hydrogel in the environment with the aqueous liquid, the self-assembled complex may absorb the aqueous liquid during swelling and release the aqueous liquid during deswelling.

In one embodiment, the aqueous liquid absorbed into the self-assembled complex may be collected and analyzed, and the aqueous liquid may include at least one of blood, plasma, serum, urine, saliva, cerebrospinal fluid, tears, sweat, feces, ascites, amniotic fluid, semen, milk, cell medium, tissue extract, and cancer tissue.

In one embodiment, the self-assembled complex may reach maximum swelling or maximum deswelling due to the isomerization of the photoswitchable isomer, and the time for the self-assembled complex to reach maximum swelling after ultraviolet light irradiation may be 30 seconds to 90 seconds, and the weight of the self-assembled complex at the time of maximum swelling may be 3 to 5 times the weight of the self-assembled complex at the time of maximum deswelling.

In one embodiment, the self-assembled complex may further include a delivery substance therein, wherein the delivery substance may be released upon swelling of the self-assembled complex, and may remain in the self-assembled complex when the self-assembled complex is in a deswollen state.

In one embodiment, the delivery substance may include at least one of a protein, a nucleic acid, and a small molecule drug, wherein the protein may include at least one of cytokines, chemokines, antibodies for anticancer immunotherapy, growth factors, botulinum toxins, and antigens, and the nucleic acid may include at least one of messenger RNA (mRNA), micro RNA, and plasmid DNA.

In one embodiment, the self-assembled complex may include one or more polymer complexes and one or more photoswitchable isomers, wherein the self-assembled complex may be formed by binding between the polymer complex and either the polymer complex adjacent thereto or the photoswitchable isomer and interaction between the photoswitchable isomer and the photoswitchable isomer adjacent thereto, and the interaction between the photoswitchable isomer and the photoswitchable isomer adjacent thereto may include at least one of π-cation interaction and π-π interaction.

In one embodiment, the photoswitchable isomer may be converted to the cis-isomer by ultraviolet light to swell the self-assembled complex, and may be converted to the trans-isomer by visible light to deswell the self-assembled complex.

In one embodiment, the polymer may include at least one of carboxymethyl cellulose, hyaluronic acid, alginate, poly(L-glutamic acid), poly(L-aspartic acid), polyacrylic acid (PAA), gelatin, and collagen.

In one embodiment, the ligand may include at least one of brassicasterol, stigmasterol, ascorbic acid, cyanocobalamin, tocopherol, retinol, aminobutyric acid, amine-Boc hydrochloride, cyclic RGD, VH 032 amide-PEG2-amine, histamine, tyramine, β-phenylethylamine, tryptamine, serotonin, putrescine, cadaverine, spermidine, and spermine.

In an embodiment, the trans-isomer of the photoswitchable isomer may have a structure represented by the following Formula 1:

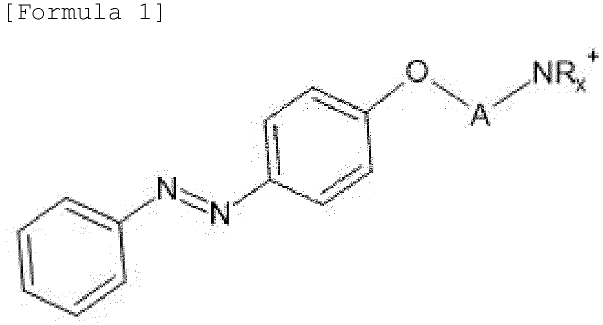

wherein A is any one of C0 to C15, R is any one of hydrogen, methyl, ethyl, and propyl, and X is an integer ranging from 1 to 3.

In one embodiment, the self-assembled complex may include the photoswitchable isomer and the polymer complex at a weight ratio of 0.1:10 to 1.0:10.

In one embodiment, the photoswitchable isomer may be converted to the cis-isomer to swell the self-assembled complex and promote macrophage adhesion to the self-assembled complex and macrophage M2 polarization, or the photoswitchable isomer may be converted to the trans-isomer to deswell the self-assembled complex, inhibit macrophage adhesion to the self-assembled complex and promote macrophage M1 polarization.

According to another aspect, embodiments of the present invention may include a light-controllable nanosystem including: the light-controllable self-assembled complex; a substrate on which at least a portion of the self-assembled complex is provided; and upconversion nanoparticles bound to the substrate, wherein the upconversion nanoparticle includes a core-shell portion and a coating portion with which the surface of the core-shell portion is coated, the core-shell portion is configured to absorb infrared light and upconvert the absorbed infrared light into at least one of ultraviolet light and visible light, and the coating portion is configured to absorb the visible light upconverted by the core-shell portion, and the upconversion nanoparticle is configured to emit the ultraviolet light upconverted by the core-shell portion.

In one embodiment, the core-shell portion may absorb light having a wavelength of 970 nm to 990 nm.

In one embodiment, the core-shell portion may include a core provided in the central portion, and a shell provided to cover the outer surface of the core, the core may include at least one of NaYbF₄, NaYF₃, NaGdF₄, KGdF, YOF, BaLaF₅, LaF₃, NaLuF₄ and SrF₂, the shell may include at least one of CaF₂, Na(Yb,Gd)F₄, NaGdF₄ and TiO₂, the core-shell portion may be doped with a dopant, and the dopant may include at least one of Yb³⁺, Tm³⁺, Ln³⁺ and Er³⁺.

In one embodiment, the coating portion may include at least one of a first coating agent and a second coating agent, the first coating agent may include at least one of β-carotene, curcumin, fluorescent protein, R-phycoerythrin, rhodamine, FDB-003, FDB-004, and FDB-005, and the second coating agent may include at least one of Tween 20, Tween 80, sodium oleate, sodium dodecyl sulphate (SDS), sodium dodecyl benzene sulphonate, sodium stearate, dodecylamine hydrochloride, span-60, span-80, polyethylene oxide, and dodecyl betaine.

In one embodiment, the first coating agent may absorb light having a wavelength of 420 nm to 500 nm.

In one embodiment, the nanosystem may work in a living body, and may be controlled by light applied from outside the living body, and when infrared light is applied to the nanosystem in the living body from outside the living body, the photoswitchable isomer in the living body may be converted to a cis-isomer to swell the self-assembled complex in the living body, and when visible light is applied to the nanosystem in the living body from outside the living body, the photoswitchable isomer in the living body may be converted to a trans-isomer to deswell the self-assembled complex in the living body.

In one embodiment, the self-assembled complex may reversibly swell and deswell in response to light in an environment with a aqueous liquid, and when the nanosystem is irradiated with infrared light, the self-assembled complex may swell and absorb the aqueous liquid, and when the nanosystem is irradiated with visible light, the self-assembled complex may deswell and release the aqueous liquid.

### BRIEF DESCRIPTION OF THE DRAWINGS

FIG. 1 schematically shows a process of regulating macrophage adhesion polarization and controlling substance delivery using a nanosystem according to one embodiment of the present invention.
FIG. 2 shows a scheme for synthesis of a photoswitchable isomer according to one embodiment of the present invention.
FIG. 3 shows the results of ¹H NMR analysis of a compound obtained in each step for a process for synthesis of the photoswitchable isomer according to one embodiment of the present invention.
FIG. 4 shows the results of analyzing the characteristics of the photoswitchable isomer according to one embodiment of the present invention.
FIG. 5 shows a scheme for synthesis of a ligand-bearing polymer according to one embodiment of the present invention.
FIG. 6 depicts a schematic view showing a mechanism for formation of a self-assembled complex according to one embodiment of the present invention, and shows the results of the number of interactions in photoswitchable isomers.
FIG. 7 shows the results of MD simulation analysis depending on isomerization of the self-assembled complex according to one embodiment of the present invention.
FIGS. 8 and 9 show the results of analyzing the characteristics of upconversion nanoparticles according to one embodiment of the present invention.
FIG. 10 shows the results of analyzing photoluminescence depending on the presence or absence of a coating portion in the upconversion nanoparticles according to one embodiment of the present invention.
FIG. 11 shows the results of an experiment conducted on the swelling and deswelling of the self-assembled complex prepared in Example 1 of the present invention.
FIGS. 12 and 13 show the results of an experiment conducted on macrophage adhesion using a nanosystem according to one embodiment of the present invention.
FIGS. 14 to 17 show the results of an experiment conducted on macrophage adhesion using the Examples of the present invention and Comparative Examples.
FIGS. 18 and 19 show the results of an experiment conducted on the time-regulated switching of swelling and deswelling of the self-assembled complex using the nanosystem according to one embodiment of the present invention.
FIGS. 20 to 23 show the results of an experiment conducted on the regulation of macrophage polarization using the nanosystem according to one embodiment of the present invention.
FIGS. 24 to 26 show the results of an experiment conducted on the control of substance delivery using the nanosystem according to one embodiment of the present invention.
FIG. 27 shows the results of an experiment conducted on the *in vivo* stability of the nanosystem according to one embodiment of the present invention.

### DETAILED DESCRIPTION

The details of other embodiments are included in the detailed description and the accompanying drawings.

The advantages and features of the present invention, and the way of attaining them, will become apparent with reference to the embodiments described below in conjunction with the accompanying drawings. However, the present disclosure is not limited to the embodiments disclosed below and may be embodied in a variety of different forms. Since all numbers, values and/or expressions referring to quantities of components, reaction conditions, etc., used in the present specification, are subject to the various uncertainties of measurement encountered in obtaining such values, unless otherwise indicated, all are to be understood as modified in all instances by the term "about." Where a numerical range is disclosed herein, such a range is continuous, inclusive of both the minimum and maximum values of the range as well as every value between such minimum and maximum values, unless otherwise indicated. Still further, where such a range refers to integers, every integer between the minimum and maximum values of such a range is included, unless otherwise indicated.

In the present specification, where a range is stated for a parameter, it will be understood that the parameter includes all values within the stated range, inclusive of the stated endpoints of the range. For example, a range of 5 to 10 will be understood to include the values 5, 6, 7, 8, 9, and 10, as well as any sub-range such as 6 to 10, 7 to 10, 6 to 9, and 7 to 9, and also include any value and range between the integers which are reasonable in the context of the range stated, such as 5.5, 6.5, 7.5, 5.5 to 8.5 and 6.5 to 9. For example, a range of "10% to 30%" will be understood to include the values 10%, 11%, 12%, 13%, etc., and all integers up to and including 30%, as well as any sub-range such as 10% to 15%, 12% to 18%, 20% to 30%, etc., and also include any value between the integers which are reasonable in the context of the range stated, such as 10.5%, 15.5%, 25.5%, etc.

The meanings of terms used in the present specification are as follows.

The term "photoswitchable isomer" refers to a compound that is converted to its trans-isomer or cis-isomer by absorbing light having a specific wavelength. The term "self-assembly" or "self-assembled complex" refers to an assembly of a ligand-bearing polymer and a photoswitchable isomer. The term "upconversion nanoparticles" means that a core-shell portion is coated with a coating portion having a function of upconversion absorbed infrared light. The term "substrate coated with upconversion nanoparticles" refers to a substrate whose surface is completely coated with the upconversion nanoparticles. The term "nanosystem" means that at least a portion of the self-assembled complex is bound to the substrate coated with the upconversion nanoparticles, and refers to a system capable of isomerizing the photoswitchable isomer by the absorption of light having a specific wavelength to regulate the uptake of a aqueous liquid, regulate cell adhesion and polarization, and regulate the release of a substance loaded in the self-assembled complex.

In addition, the terms "light" and "light energy" may be used with the same meaning. Furthermore, the term "deswell" or "deswelling" and the term "shrink" or "shrinking" are used interchangeably with each other.

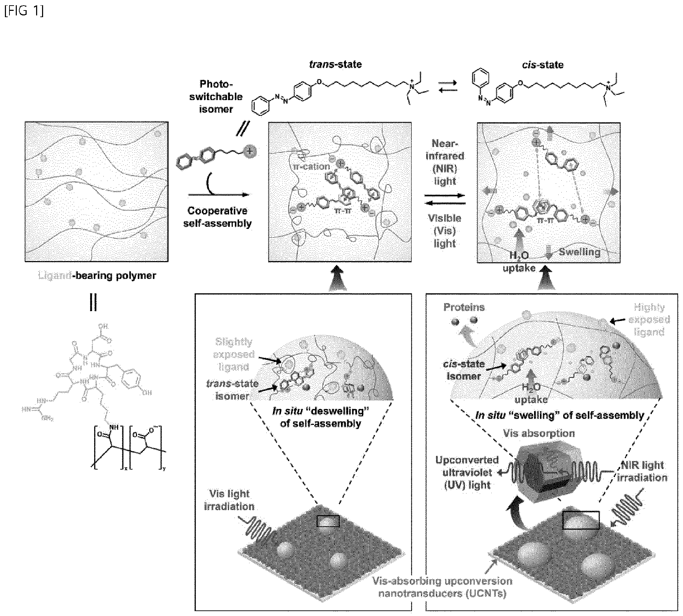

FIG. 1 schematically shows a process of regulating macrophage adhesion and polarization and regulating the release of a substance loaded in the self-assembled complex by irradiating the nanosystem according to the present invention with light.

An embodiment of the present invention is directed to a light-controllable self-assembled complex including: a polymer complex including a ligand and a negatively charged polymer; and a positively charged photoswitchable isomer which is bound to the polymer complex and is reversibly isomerized by light. The photoswitchable isomer may be isomerized by light to reversibly swell or deswell the self-assembled complex. In addition, the cis-isomer of the photoswitchable isomer may swell the self-assembled complex, and the trans-isomer of the photoswitchable isomer may shrink the self-assembling complex.

The self-assembled complex may include one or more polymer complexes and one or more photoswitchable isomers, wherein the self-assembled complex may be formed by bonding between the polymer complex and the polymer complex adjacent thereto or the photoswitchable isomers and interaction between the photoswitchable isomer and the photoswitchable isomer adjacent thereto

Specifically, the self-assembled complex may be formed by bonding or interaction between a plurality of polymer complexes and a plurality of photoswitchable isomers. For example, a physical or chemical bond may be formed between the polymer complexes constituting the self-assembled complex, and an electrostatic bond may be formed between the polymer complex and the photoswitchable isomer, and the self-assembled complex may be formed by interaction between the photoswitchable isomers.

The swelling and deswelling of the self-assembled complex may occur while the interaction between the photoswitchable isomers is altered.

The photoswitchable isomer may include an aromatic functional group, and the photoswitchable isomers may interact with each other via the aromatic functional group.

The interaction between the photoswitchable isomers may be said to occur between one photoswitchable isomer and a photoswitchable isomer adjacent thereto. As used herein, the term "adjacent" means that other photoswitchable isomers are present around any photoswitchable isomer.

The interaction between the photoswitchable isomers may be a π-cation interaction between the aromatic functional group of any one photoswitchable isomer and the cation of other photoswitchable isomer. In addition, the interaction between the photoswitchable isomers may be a π-π interaction between the aromatic functional group of any one photoswitchable isomer and the aromatic functional group of other photoswitchable isomer.

The aromatic functional group of the photoswitchable isomer may be a benzene functional group. The photoswitchable isomer may exist as any one of a trans-isomer and a cis-isomer, and the trans-isomer may be a more stable form. The cis-isomer of the photoswitchable isomer may be isomerized to the trans-isomer by absorbing visible light. In addition, the trans-isomer may be isomerized to the cis-isomer by absorbing ultraviolet light. This switching between the trans and cis-isomers may be reversibly repeated and may occur rapidly.

The trans-isomer of the photoswitchable isomer may have a structure represented by the following Formula 1:

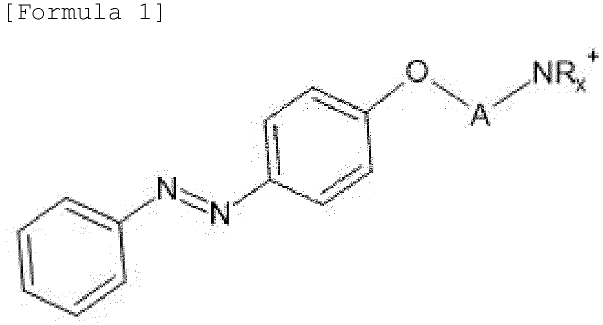

wherein A is any one of C0 to C15, R is any one of hydrogen, methyl, ethyl, and propyl, and X is an integer ranging from 1 to 3. Preferably, X may be 3. Specifically, A may be C1 to C15, or C1 to C10.

In addition, the cis-isomer of the photoswitchable isomer may have a structure represented by the following Formula 2: 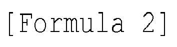 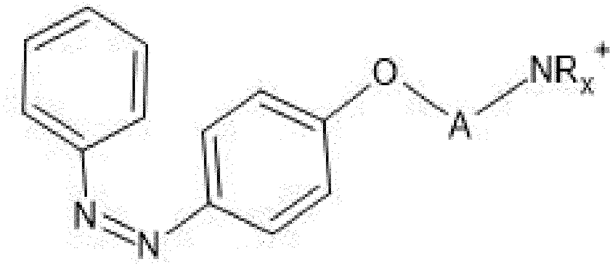 wherein is any one of C0 to C15, R is any one of hydrogen, methyl, ethyl, and propyl, and X is an integer ranging from 1 to 3. X may preferably be 3. Specifically, A may be C1 to C15, or C1 to C10.

The self-assembled complex may include the photoswitchable isomer and the polymer complex at a weight ratio of 0.1:10 to 1.0:10. The self-assembled complex can reversibly swell and deswell in response to light, and the extent and/or speed of swelling and deswelling can be variously changed by the weight ratio between the photoswitchable isomer and the polymer complex.

In the polymer complex, the polymer may form a framework for the self-assembled complex. When the self-assembled complex shrinks, the polymer complex may be condensed. In addition, when the self-assembled complex swells, the polymer complex may be decondensed. Since the polymer includes a negatively charged site, it may provide a site to which the photoswitchable isomer can electrostatically bind. The polymer may have a carboxylate group.

The polymer may be at least one of carboxymethyl cellulose, hyaluronic acid, alginate, poly(L-glutamic acid), poly(L-aspartic acid), polyacrylic acid (PAA), gelatin, and collagen.

In the polymer complex, the ligand may exist bound to the polymer. When the self-assembled complex shrinks, the polymer may be coiled and condensed so that most of the ligand may be present inside the self-assembled complex, and the ligand may be difficult to recognize from the outside of the self-assembled complex. Thus, the ligand exposed to the outside of the self-assembled complex may be very small. Conversely, when the self-assembled complex swells, the polymer may be uncoiled, and at least some of the polymer molecules may be spaced apart from each other so that most of the ligand is exposed to the outside of the self-assembled complex, and the ligand may be easily recognized from the outside of the self-assembled complex. Thus, in this case, the ligand exposed to the outside of the self-assembled complex may relatively increase.

The ligand has cell adhesion properties and may be a site which is recognized by cells for adhesion. Thus, when the self-assembled complex swells, the number and density of the ligands, which are recognized by cells provided outside the self-assembled complex, may increase, and thus adhesion of the cells may increase. Conversely, when the self-assembled complex shrinks, the number and density of the ligands, which are recognized by cells provided outside the self-assembled complex, may decrease, and thus adhesion of the cells may decrease.

The ligand may contain at least one of a hydroxyl group (-OH) and an amino group (-NR₃).

The ligand may include at least one of brassicasterol, stigmasterol, ascorbic acid, cyanocobalamin, tocopherol, retinol, aminobutyric acid, amine-Boc hydrochloride, cyclic RGD, VH 032 amide-PEG2-amine, histamine, tyramine, β-phenylethylamine, tryptamine, serotonin, putrescine, cadaverine, spermidine, and spermine.

The self-assembled complex may be formed in the form of a frame by the polymer complex, and the frame may have an internal space. A delivery material may be loaded in the inner space of the frame.

The delivery substance may be loaded in the self-assembled complex and delivered to the site of action of the delivery substance. The delivery substance may be released out of the self-assembled complex when the self-assembled complex swells, and may not be released when the self-assembled complex is in a shrunk state. Therefore, when the self-assembled complex shrinks, the delivery substance may be maintained inside the self-assembled complex. The delivery substance may be continuously released for a certain period of time when the self-assembled complex swells.

The delivery material may be included at a weight ratio of 1:100 to 1:1,000 relative to the polymer complex. For example, in the self-assembled complex according to this embodiment, the amount and/or type of delivery substance loaded in the self-assembled complex can be variously changed by controlling the weight ratio between the delivery substance and the polymer complex within the above-described range, and the release rate of the delivery substance can also be controlled.

The delivery substance may include at least one of a protein, a nucleic acid, and a small molecule drug. Specifically, the protein may include at least one of cytokines, chemokines, antibodies for anticancer immunotherapy, growth factors, botulinum toxin, and antigens. In addition, the nucleic acid may include at least one of messenger RNA (mRNA), micro RNA (miRNA), and plasmid DNA.

More specifically, the delivery substance may be a cytokine, and may preferably be at least one of ANG, BDNF, CCL1, CCL11, CCL13, CCL15, CCL17, CCL18, CCL2, CCL20, CCL22, CCL23, CCL24, CCL26, CCL4, CCL5, CCL7, CCL8, CSF1, CSF2, CSF3, CX3CL1, CXCL1, CXCL10, CXCL12, CXCL13, CXCL2, CXCL3, CXCL5, CXCL6, CXCL8, CXCL9, EGF, FGF4, FGF6, FGF7, FGF9, FLT3LG, GDNF, HGF, IFNG, IGF1, IGFBP1, IGFBP2, IGFBP3, IGFBP4, IL10, IL12A, IL13, IL15, IL16, IL1A, IL1B, IL2, IL3, IL4, IL5, IL6, IL7, KITLG, LEP, LIF, LTA, MIF, NTF3, NTF4, OSM, PDGFB, PGF, PPBP, SPP1, TGFB1, TGFB2, TGFB3, THPO, TIMP1, TIMP2, TNF, TNFRSF11B, TNFSF14, TPO, VEGFA, and granulocyte-macrophage colony-stimulating factor (GM-CSF).

More specifically, the delivery substance may also be a chemokine, and may preferably be at least one of IFN gamma, IFNL1, AXL, BTC, CCL13, CCL14, CCL16, CCL17, CCL18, CCL19, CCL20, CCL21, CCL22, CCL23, CCL25, CCL26, CCL27, CCL28, CCL4, CCL7, CCL8, CXCL1, CXCL10, CXCL11, CXCL12, CXCL16, CXCL2, CXCL3, CXCL5, and CXCL6.

More specifically, the delivery substance may also be an antibody for anticancer immunotherapy, and may preferably be at least one of an anti-PD-1 antibody and an anti-PD-Ll antibody.

More specifically, the delivery substance may also be growth factor, and may preferably be at least one of vascular endothelial growth factor (VEGF), bone morphogenetic protein (BMP), transforming growth factor-β (TGF-β), and insulin-like growth factor (IGF).

More specifically, the delivery substance may also be a small molecule drug, and may preferably be at least one of dexamethasone, vitamin B3, vitamin C, doxolubicin, adenosine, triamcinolone, non-steroidal anti-inflammatory drugs (NSAIDs), allantoin, and gentamicin.

The self-assembled complex may reversibly swell and deswell by light irradiation in an environment with a aqueous liquid. When the self-assembled complex swells, it may absorb the aqueous liquid, and when the self-assembled complex deswells, it may release the aqueous liquid. Therefore, the self-assembled complex may be present in the form of a hydrogel by absorbing the aqueous liquid. For example, hydrogels formed from the self-assembled complexes may be combined together to form a larger hydrogel.

When the self-assembled complex swells, it may absorb the aqueous liquid, and when the self-assembled complex deswells, it may release the aqueous liquid. The self-assembled complex may behave in an environment with a aqueous liquid, that is, may behave in a hydrophilic environment. Accordingly, the surroundings of the self-assembled complex may include a aqueous liquid, and the self-assembled complex may absorb or release the aqueous liquid from or to the surroundings.

In addition, the inside of the self-assembled complex may contain some aqueous liquid even in a deswollen state. Thus, even when the self-assembled complex swells to absorb the aqueous liquid and then deswells to release the aqueous liquid, the self-assembled complex may contain some aqueous liquid therein.

When the self-assembled complex absorbs or releases the aqueous liquid due to the isomerization of the photoswitchable isomer, the self-assembled complex may reach maximum swelling or maximum deswelling. As used herein, the term "maximum swelling and maximum deswelling" means that the self-assembled complex swells or deswells due to the isomerization of the photoswitchable isomer and/or the absorption or release of the aqueous liquid within a range in which the bond or interaction in the self-assembled complex is maintained. Switching between the maximum swelling and minimum swelling of the self-assembled complex may occur at very high speed. For example, the self-assembled complex may reach maximum swelling within 30 seconds to 90 seconds after irradiation with ultraviolet light in the minimum deswelling state.

In addition, the maximum swelling of the self-assembled complex may increase the weight by the absorbed aqueous liquid. The weight of the self-assembled complex at the time of maximum swelling may be 3 to 5 times the weight at the time of maximum deswelling.

For example, the photoswitchable isomer of the self-assembled complex may be isomerized to the cis-isomer by absorption of ultraviolet light, and while the self-assembled complex swells, the aqueous liquid may be absorbed into the self-assembled complex. In addition, for example, when the photoswitchable isomer of the self-assembled complex is isomerized to the trans-isomer by absorption of visible light, and while the self-assembled complex swells, the aqueous liquid inside the self-assembled complex may be released.

Specifically, when the photoswitchable isomer absorbs ultraviolet light by irradiating the self-assembled complex with light, cis-isomerization may occur. In the cis-isomer, a π-π interaction may be formed well, but a π-cation interaction may be difficult to form. As the interaction formed in the cis-isomer is weakened, the self-assembled complex may swell, and at the same time, the surrounding aqueous liquid may be absorbed into the self-assembled complex. In addition, in this case, where a delivery substance is loaded in the self-assembled complex, the delivery substance may be released, and a large amount of the ligand attached to the polymer complex may be exposed to the outside.

In addition, specifically, when the photoswitchable isomer absorbs visible light by irradiating the self-assembled complex with light, trans-isomerization may occur. In the trans photoswitchable isomer, both π-cation and π-π interactions may be formed. As the interactions formed in the photoswitchable isomer becomes stronger, the self-assembled complex may deswell, and at this time, the aqueous liquid inside the self-assembled complex may be released to the surrounding environment. In this case, where a delivery substance is loaded in the self-assembled complex, the release of the delivery material may be suppressed, and the ligand attached to the polymer complex may hardly be exposed to the outside.

When the aqueous liquid is absorbed into the self-assembled complex, the absorption of the aqueous liquid may help the self-assembled complex to swell. In addition, when the aqueous liquid inside the self-assembled complex is released, the release of the aqueous liquid may help the self-assembled complex to deswell.

The aqueous liquid may include at least one of water, water-soluble molecules, and biomolecules that may exist in body fluids *in vivo.* The aqueous liquid may include any one or more of blood, plasma, serum, urine, saliva, cerebrospinal fluid, tears, sweat, feces, ascites, amniotic fluid, semen, milk, cell medium, tissue extract, and cancer tissue.

Based on the reversible swelling and deswelling characteristics of the self-assembled complex as described above, the self-assembled complex may be implanted into a living body.

The self-assembled complex may be implanted into sites such as skin, tissue and organs. At this time, the self-assembled complex may be manipulated to absorb the aqueous liquid in the corresponding site by irradiating the self-assembled complex with light, and the self-assembled complex may be recovered and the absorbed aqueous liquid may be extracted and used as a biological sample. The biological sample may be used for disease testing, cell analysis, and the like.

In addition, the self-assembled complex may be implanted into an *in vivo* vessel, such as a blood vessel or lymphatic vessel. At this time, the self-assembled complex can be manipulated to absorb the aqueous liquid at the site by irradiating light. At this time, the self-assembled complex may be manipulated to absorb the aqueous liquid at the corresponding site by irradiating light. In addition, when the self-assembled complex is allowed to swell in a vessel by irradiating light, it is possible to block the flow of liquid in the vessel or inhibit the growth of the vessel. For example, when the self-assembled complex is allowed to swell in a blood vessel, it is possible to inhibit the growth of cancer cells. In this case, when an anticancer drug is loaded in the self-assembled complex, cancer treatment may be induced.

In addition, the self-assembled complex may be loaded with the delivery substance and implanted into a living body. In this case, it is possible to regulate the release of the delivery substance by irradiating the self-assembled complex with light. Therefore, the delivery substance may be directly and quickly delivered to a desired site using the self-assembled complex.

The self-assembled complex may also be implanted together with cells such as macrophages. In this case, it is possible to regulate the adhesion and polarization of macrophages at the implanted site by irradiating the self-assembling complex with light. The self-assembled complex may be provided inside or outside the living body so that the volume thereof may reversibly swell or shrink. The light may be applied to the self-assembled complex from inside or outside the living body. Specifically, where the self-assembled complex is inside the living body, even when the light is applied thereto from outside the living body, the volume of the self-assembled complex may reversibly swell or shrink.

In one embodiment of the present invention, a nanosystem that behaves depending on light irradiation may include: the self-assembled complex that behaves depending on light irradiation; a substrate to which at least a portion of the self-assembled complex is bound; and upconversion nanoparticles bound to the substrate. The upconversion nanoparticle may include a core-shell portion, and a coating portion with which the surface of the core-shell portion is coated. The core-shell portion may be configured to absorb infrared light and upconvert the absorbed infrared light into at least one of ultraviolet light and visible light, and the coating portion may be configured to absorb the visible light upconverted by the core-shell portion, and the upconversion nanoparticle may be configured to emit the ultraviolet light upconverted by the core-shell portion.

The core-shell portion may include a core provided in the central portion, and a shell provided to completely cover the outer surface of the core. The core-shell portion may absorb light having a specific wavelength and upconvert the absorbed light.

For example, the core-shell portion may absorb infrared light and convert the absorbed light into ultraviolet light. The infrared light absorbed in this process may be converted into at least one of visible light and ultraviolet light.

The core-shell portion may absorb light having a wavelength of 970 nm to 990 nm.

In the core-shell portion, the core may include at least one of NaYbF₄, NaYF₃, NaGdF₄, KGdF, YOF, BaLaF₅, LaF₃, NaLuF₄ and SrF₂. The shell may include at least one of CaF₂, Na(Yb,Gd)F₄, NaGdF₄ and TiO₂.

In addition, the core may be doped with a dopant, and the dopant may include at least one of Yb³⁺, Tm³⁺, Ln³⁺ and Er³⁺. Preferably, among the above dopants, at least one of Yb³⁺ and Tm³⁺ may be essentially used for doping.

The core-shell portion may be coated with a coating portion covering the entire outer surface of the core-shell portion. The coating portion may absorb visible light among the light converted by the core-shell portion and may not absorb ultraviolet light. In other words, among the light converted from infrared rays absorbed by the core-shell portion, visible light may be absorbed by the coating portion and only ultraviolet light may be emitted to the outside. Thus, the coating portion of the upconversion nanoparticle may absorb infrared light and selectively emit ultraviolet light. Thus, the coating portion may absorb light having a wavelength of 420 nm to 500 nm, and may not absorb light having a wavelength of 320 nm to 380 nm.

The coating portion may be formed by mixing a first coating agent and a second coating agent. The first coating agent may absorb visible light upconverted by the core-shell portion, may not absorb ultraviolet light, and may preferably be a dye. The second coating agent may help the first coating agent to be stably coated, and may preferably be a surfactant. The second coating agent may be configured to cover the surface of the first coating agent.

**The** first coating agent may be a dye and may include at least one of β-carotene, curcumin, R-phycoerythrin, rhodamine, FDB-003, FDB-004, and FDB-005.

The second coating agent may be a surfactant, and may include at least one of a cationic surfactant, an anionic surfactant, a nonionic surfactant, and an amphoteric surfactant.

The second coating agent may include a cationic surfactant. Specifically, it may include at least one of sodium oleate, sodium dodecyl sulfate (SDS), sodium dodecyl benzene sulfonate, and sodium stearate.

In addition, the second coating agent may include an anionic surfactant. Specifically, it may include dodecylamine hydrochloride.

In addition, the second coating agent may include a nonionic surfactant. Specifically, it may include at least one of Tween 20, Tween 80, span-60, span-80, and polyethylene oxide.

In addition, the second coating agent may include an amphoteric surfactant. Specifically, it may be dodecyl betaine.

In the nanosystem that is controlled by light, the upconversion nanoparticles may be bound to the substrate, and the self-assembled complex may be bound to at least one of the substrate and the upconversion nanoparticles.

The photoswitchable isomer may be isomerized to the cis-isomer by irradiating the nanosystem with infrared light. In this case, the volume of the self-assembled complex may reversibly swell or shrink.

For example, when the self-assembled complex swells, a aqueous liquid may be absorbed into the self-assembled complex, and where a delivery substance is loaded in the self-assembled complex, the delivery substance may be released out of the self-assembled complex. Infrared light with which the nanosystem has been irradiated may be upconverted to ultraviolet light through the upconversion nanoparticles, and the photoswitchable isomer may be isomerized to the cis-isomer by absorbing the upconverted ultraviolet light.

Alternatively, the photoswitchable isomer may be isomerized to the trans-isomer by irradiating the nanosystem with visible light. In this case, the self-assembled complex may deswell, and the aqueous liquid inside the self-assembled complex may be released. Visible light with which the nanosystem has been irradiated is not absorbed by the upconversing nanoparticles, but may be directly absorbed by the photoswitchable isomer to induce trans-isomerization.

Meanwhile, the self-assembled complex may swell expand to absorb the water-aqueous liquid and release the delivery substance loaded therein. Alternatively, the self-assembled complex may deswell to release the aqueous liquid, and in this case, the delivery substance may not be released.

In addition, the self-assembled complex may include a cell-adhesive ligand therein, and thus may regulate cell adhesion and polarization. The cells may be macrophages.

When the self-assembled complex is irradiated with UV light, the photoswitchable isomer may be isomerized to the cis-isomer by absorbing the UV light, so that the self-assembled complex may swell. In this case, a large amount of the ligand may be exposed to the outside of the self-assembled complex, and may promote macrophage adhesion and M2 polarization.

Alternatively, when the self-assembled complex is irradiated with visible light, the photoswitchable isomer may be isomerized to the trans-isomer, and the self-assembled complex may deswell. In this case, the ligand is hardly exposed to the outside of the self-assembled complex, and may inhibit macrophage adhesion and promote macrophage M1 polarization.

Where the self-assembled complex forms the nanosystem together with the substrate and the upconversing nanoparticles, it is possible to promote macrophage adhesion and M2 polarization by irradiating the nanosystem with infrared light, and it is possible to inhibit macrophage adhesion and promote macrophage M1 polarization by irradiating the nanosystem with visible light.

Such infrared or visible light may be irradiated from outside the living body and act on the nanosystem inside the living body.

Although ultraviolet light isomerizes the photoswitchable isomer to the cis-isomer, ultraviolet light may have problems in that it has high *in vivo* toxicity and low *in vivo* permeability. In addition, when the photoswitchable isomer is used without using upconversion nanoparticles or when a conventional upconversing material is used, a problem may arise in that it is necessary to irradiate ultraviolet light harmful to the living body. On the other hand, infrared and visible light with which the nanosystem is directly irradiated is harmless to the living body and can control the behavior of the nanosystem including the upconversion nanoparticles. Thus, when the nanosystem include the upconversion nanoparticles, it is possible to control the nanosystem without directly irradiating ultraviolet light.

Therefore, in the case of the nanosystem, the behavior of the nanosystem inside the living body may be controlled by irradiating the nanosystem with harmless infrared and visible light from outside the living body, thereby controlling the absorption of the aqueous liquid, regulating substance delivery, and regulating macrophage adhesion and polarization.

Hereinafter, examples of the present invention and comparative examples will be described. However, the following examples are only preferred examples of the present invention, and the scope of the present invention is not limited by the following examples.

### [Preparation Example]

### 1. Synthesis of photoswitchable isomer

As a photoswitchable isomer that reversibly regulates the swelling and deswelling of a self-assembled complex, Azo-C10-N+ was synthesized.

First, 3.00 g of 4-aminophenol (27.49 mmol) and 3.24 g of nitrosobenzene (30.24 mmol) were mixed with 100 mL of acetic acid in a two-neck round-bottom flask, and then stirred overnight at room temperature under N₂, thereby synthesizing Azo-OH. DI water was added to the mixture solution to obtain a reddish-brown colored precipitate. The precipitate was collected using ethyl acetate (EA) and dried with MgSO₄, MgSO₄ was removed by filtration, and EA was removed by evaporation. Finally, gel column chromatography was performed using hexane/ethyl acetate (6:1 (v/v)) as an eluent.

To facilitate the interaction between the aromatic group and the cation of the Azo-C10-N+ molecule in a self-assembled complex, a gap was given between the aromatic group and Br by forming a hydrophobic tail. To obtain this Azo-C10-Br molecule, 1,10-dibromodecane was bound to Azo-OH. Specifically, 0.5 g of Azo-OH (2.52 mmol) was dissolved in 40 mL of dimethylformamide (DMF), and 40 mL of sodium hydride (5.04 mmol) was added thereto in an ice bath. The solution was stirred for 30 minutes, and 5 mL of DMF containing 3.78 g of 1,10-dibromodecaine (12.60 mmol) was added thereto, followed by stirring at room temperature overnight. Thereafter, the reaction was quenched by adding 5 mL of methanol to the solution, and the synthesized Azo-C10-Br was extracted with a mixture of DI water and dichloromethane. Azo-C10-Br dissolved in dichloromethane was dried with MgSO₄ to remove DI water, and then 1:4 (v/v) dichloromethane/hexane was included as an eluent, and MgSO₄ was removed by filtration using gel column chromatography using dichloromethane/hexane (1:4 (v/v)) as an eluent, and dichloromethane was removed by evaporation.

Then, an Azo-C10-N+ molecule was produced in which Br in the Azo-C10-Br molecule was replaced with triethylamine having a nitrogen cation and which had a gap between the aromatic group and the nitrogen cation due to a hydrophobic tail. These molecules were stacked together through π-cation interaction and π-π interaction to form a self-assembled complex containing a ligand. Briefly, a solution of 0.3 g of Azo-C10-Br (0.72 mmol) and 0.87 g of triethylamine (8.63 mmol) in 40 mL of acetonitrile was stirred overnight at 60°C under N₂. After evaporating acetonitrile, the resulting yellow solid product was dissolved in tetrahydrofuran (THF) to remove excess trimethylamine. Hexane was added to the solution to precipitate the Azo-C10-N+ product, which was then filtered and dried in a vacuum oven.

### 2. Synthesis of ligand-bearing polymer

In order to bind a cell-adhesive RGD ligand to a self-assembled complex, polyacrylic acid (PAA), a positively charged polymer, a ligand-bearing and negatively charged polymer, was electrostatically bound to the positively charged Azo-C10-N+ molecule stack. Here, as the RGD ligand, cyclic-RGDyK (cyclo-RGDyK, cyclo-Arg-Gly-Asp-d-Tyr-Lys) with a lysine residue bearing an amino group was used.

Through a BOP [(benzotriazol-1-yloxy)tris(dimethylamino)phosphonium hexafluorophosphate]-mediated amide linkage between the amino group of RGDyK and the carboxylate group of PAA, the RGD ligand was connected to PAA. Briefly, 116 mg of PAA (Mw=1800 Da, 0.065 mmol), 10 mg of cyclo-RGDyK (RGD ligand peptide, AnaSpec Inc., 0.016 mmol), 57.1 mg of BOP (0.129 mmol), and 17.4 mg of hydroxybenzotriazole hydrate (HOBt, 0.129 mmol) were mixed together in 2mL of DMF containing N,N-diisopropylethylamine (DIPEA, 0.258 mmol).

The mixture was vigorously stirred overnight at room temperature under N₂. To selectively collect the ligand-bearing PAA, the product was dialyzed against DI water using a dialysis bag having a molecular weight cut-off of 500 Da.

### 3. Preparation of self-assembled complex

For reversible photoswitching between swelling and deswelling of a ligand-bearing self-assembled complex, 3 mL of phosphate-buffered saline (PBS) containing the ligand-bearing PAA (16.8 µmol), and 3 mL of PBS containing Azo-C10-N+ (Mw=438.7Da, 3.42 µmol) were mixed together by sonication for 10 minutes, thereby preparing a self-assembled complex in which the ligand-bearing PAA and Azo-C10-N+ were connected to each other via electrostatic interaction.

Thereafter, the solution was briefly centrifuged at 1,000 rpm for 10 minutes, large precipitates were removed, and the supernatant containing a uniform ligand-bearing self-assembled complex was collected.

### 4. Synthesis of upconversion nanoparticles

To control the swelling and deswelling of nanoassemblies by dual-wavelength photostimulation (NIR and Vis light) without UV light (and thus relatively tissuepenetrating), upconversion nanoparticles (upconversion nanotransducers (UCNTs)) were synthesized.

### (1) Preparation of core-shell portion

The core-shell portion (NaRF₄: Rb, Tm@NaYF₄) of the upconversion nanoparticle was prepared. Through conformal shell coating on the core of the core-shell portion, the surface of the core-shell portion was passivated to limit the extinction of photoluminescence generated from surface defects. This surface passivation allowed to upconvert the irradiated NIR and to emit UV light with high photoluminescence intensity.

The 49% Yb- and 1% Tm-doped NaYF₄ core in the core-shell portion was prepared by mixing 0.5 mmol yttrium (III) chloride hexahydrate (YCl₃·6H₂O, 99.99%), 0.49 mmol ytterbium (III) chloride hexahydrate (YbCl₃·6H₂O, 99.99%), 1 mmol thulium (III) chloride hexahydrate (TmCl₃·6H₂O, 99.99%), 6 mL oleic acid (90%), and 15 mL octadecene (90%) together in a three-neck flask, followed by heating at 150°C for 40 minutes. Thereafter, 10 mL of methanol containing 2.5 mmol NaOH and 4 mmol NH₄F was added to the mixture solution, followed by stirring for 40 minutes. Next, methanol was evaporated out, and the mixture solution was heated at 320°C under an Ar atmosphere for 60 minutes. The mixture solution was washed three times with ethanol, and the synthesized core (NaYF₄:Yb, Tm) was dispersed in 10mL of cyclohexane.

The NaYF₄ shell was formed by epitaxial growth on the core to minimize surface defects and increase photoluminescence intensity. Briefly, 1 mmol YCl₃·6H₂O, 6 mL oleic acid and 15 mL 1-octadecene were mixed together in a three-necked flask to make a mixture solution which was then heated at 150°C for 40 minutes. Thereafter, 10 mL of the core suspension prepared as described above and a methanol solution containing 2.5 mmol NaOH and 4 mmol NH₄F were sequentially added to the mixture solution, followed by stirring for 40 minutes. After methanol was evaporated out, the mixture solution was heated to 320°C for 60 minutes under an Ar atmosphere, and then washed three times with ethanol.

Finally, the prepared core-shell portion (NaYF₄:Yb, Tm@NaYF₄) was dispersed in 10 mL of cyclohexane.

### (2) Formation of coating portion on core-shell portion

In order to induce selective isomerization by irradiating the self-assembled complex with light, a coating part was prepared to upconvert NIR light to both Vis light and UV light to the maximum extent after irradiating the upconversion nanoparticles with NIR. To this end, the surface of the core-shell portion prepared in (1) above was coated with the natural dye β-carotene absorbing visible light in the range of 400 nm to 500 nm, and this coating portion absorbed upconverted visible light capable of isomerizing the photoswitchable isomer to the trans-isomer.

A solution of 500 µL of the core-shell portion (10 mg/mL), 100 µL of the clinically used natural dye β-carotene (1 mg/mL), and 200 µL of clinically used Tween 80 (10 mg/mL) in cyclohexane was vigorously vortexed. The resulting solution was air-dried to evaporate cyclohexane, and the dried residue was dispersed in 2 mL of DI water. Thereafter, the dispersion was continuously sonicated, and stirred for 2 minutes such that the formed upconversion nanoparticles (core@shell@coating portion) did not agglomerate.

The obtained upconversion nanoparticles were stored in the dark before use.

### (3) Formation of substrate coated with upconversion nanoparticles

The entire surface of a glass substrate was coated with the upconversion nanoparticles prepared in (2) above.

Cell culture-grade glass substrates and silicon substrates (having surface chemistry similar to that of glass substrates due to a natural silicon oxide surface formed thereon) were cut into a size of 12 mm X 12 mm and used in experiments. The substrates were immersed in HCl for 30 minutes to clean the surfaces, and were treated with H₂SO₄ for 1 hour to activate the hydroxyl group. Thereafter, the substrates were rinsed three times with DI water and incubated in DI water containing 400 µL of the upconversion nanoparticles to coat the surface of the substrate with upconversion nanoparticles. Next, the substrates were dried in a drying oven at 40°C for 6 hours.

The surfaces of the upconversion nanoparticles were uniformly dispersed by hydrophilic interactions with the hydroxyl groups on the substrate surface, and the upconversion nanoparticles completely covered the substrate surface.

The substrates coated with these upconversion nanoparticles were further coated with DI water containing 400 µL of the natural dye β-carotene, and then completely dried in an oven at 40°C for 6 hours.

The substrates thus prepared were sterilized with UV light for 1 hour before use in cell culture.

### 5. Preparation of nanosystem

A nanosystem was prepared by combining the self-assembled complex prepared above and the substrate coated with the upconversion nanoparticles.

The substrate coated with the upconversion nanoparticles was incubated in phosphate-buffered saline (PBS) containing 1 mL of the self-assembled complex for 30 minutes at room temperature, and then washed with Dulbecco's phosphate-buffered saline (D-PBS).

In this process, due to the hydrophilic interaction between the upconversion nanoparticles and the self-assembled complex, the self-assembled complex was bound to the substrate coated with the upconversion nanoparticles and formed a spread shape. Therefore, a nanosystem capable of reversibly swelling and deswelling the self-assembled complex by changing the isomeric form of the photoswitchable isomer through light irradiation was prepared.

### [Examples]

According to the Preparation Example, Examples were carried out.

### 1. Example 1

A self-assembled complex was formed by electrostatically binding a photoswitchable isomer having an Azo-C10-N+ structure to an RGD ligand-bearing PAA polymer.

In addition, the surface of a substrate was completely coated with upconversion nanoparticles having an NaYF₄:Yb, Tm@NaYF₄@ coating portion through hydrophilic interaction. As the dye forming the inner layer of the coating portion, β-carotene was used, and the surface of the inner layer was completely coated with the surfactant Tween 80 to form the outer layer of the coating portion.

The self-assembled complex was attached to the outer layer of the upconversion nanoparticle by hydrophilic interaction.

The inside of the self-assembled complex was loaded with β-carotene as a dye.

### 2. Example 2

Example 2 was performed in the same manner as in Example 1, except that rhodamine was used instead of β-carotene as the substance loaded in the self-assembled complex.

### 2. Example 3

Example 3 was performed in the same manner as in Example 1, except that IL-4 was used instead of β-carotene as the substance loaded in the self-assembled complex.

### [Comparative Examples]

### 1. Comparative Example 1

A material having only the substrate without the self-assembled complex and the upconversion nanoparticles was prepared in the same manner as in Example 1.

### 2. Comparative Example 2

A material having the upconversion nanoparticles and the self-assembled complex on the substrate was prepared in the same manner as in Example 1, except that the upconversion nanoparticle had only the core without the shell and the coating portion.

### 3. Comparative Example 3

A material having the upconversion nanoparticles and the self-assembled complex on the substrate was prepared in the same manner as in Example 1, except that the upconversion nanoparticle had only the core-shell portion without the coating portion.

### 4. Comparative Example 4

A material having the self-assembled complex on the substrate was prepared in the same manner as in Example 1, except that the upconversion nanoparticle was not provided on the substrate.

### 5. Comparative Example 5

A material having the upconversion nanoparticles on the substrate was prepared in the same manner as in Example 1, except that the self-assembled complex was not provided on the substrate.

### 6. Comparative Example 6

A material having the self-assembled complex and the upconversion nanoparticles on the substrate was prepared in the same manner as in Example 1, except that the self-assembled complex did not have the ligand.

### [Experimental Methods]

### 1. ¹H NMR spectrometry

¹H NMR spectroscopy (Bruker Advance 400 spectrometer, 400 MHz) was used to analyze changes in a series of chemical bonds in Azo-OH, Azo-C10-Br, and Azo-C10-N+ molecules.

Prior to analysis, Azo-OH was dissolved in deuterated dimethyl sulfoxide (DMSO-d6), and Azo-C10-Br and Azo-C10-N+ were dissolved in deuterated chloroform (CDCl₃). Characteristic peaks were indexed after synthesis of each sample.

The Azo-OH sample was yellow-orange in color (1.4 g, 25.7% yield) . ¹H NMR data of Azo-OH (400 MHz, DMSO-d6, δ (ppm)) were as follows: 10.34 (s, 1H), 7.84-7.82 (m, 4H) 7.59-7.49 (m, 3H), 6.99-6.95 (m, 2H). A phenolic group was identified as a characteristic peak of the Azo-OH molecule.

The Azo-C10-Br molecule sample was yellow-orange color in color (0.5 g, yield: 47.6%). ¹H NMR data of Azo-C10-Br (400 MHz, CDCl₃, δ (ppm)) were as follows: 7.93-7.85 (m, 4H), 7.52-7.41 (m, 3H), 7.00-6.99 (d, J = 9.2 Hz, 2H), 4.05-4.02 (t, J = 6.4 Hz, 2H), 3.42-3.39 (t, J = 7.2 Hz, 2H), 1.89-1.78 (m, 4H), 1.51-1.38 (m, 4H), 1.32 (s, 8H). An ether group was identified as a characteristic peak of the Azo-C10-Br molecule.

The Azo-C10-N+ molecule sample was yellow in color (0.24 g, yield: 64.9%). ¹H NMR data of Azo-C10-N+ (400 MHz, CDCl₃, δ (ppm)) were as follows: 7.92-7.86 (m, 4H), 7.52-7.42 (m, 3H), 7.02-6.99 (d, J = 8.8 Hz, 2H), 4.06-4.03 (t, J = 6.4 Hz, 2H), 3.54-3.46 (m, 6H), 3.29-3.25 (m, 2H), 1.49-1.33 (m, 25H). α-hydrogen of a tertiary amine was identified as a characteristic peak of the Azo-C10-N+ molecule.

### 2. Molecular dynamic simulation

In order to confirm that the amount of water absorption of cis-Azo-C10-N+ is higher than that of trans-Azo-C10-N+, the affinity for each Azo-C10-N+ for water was determined by calculating hydration energy using Schrödinger Suites (Schrödinger).
(1) The preliminary conformers of the molecules were searched by MacroModel with the following parameters:
   1) force field : OPLS3;
   2) solvent: H₂O;
   3) charged from force field;
   4) cutoff: none;
   5) minimization method: PRCG;
   6) maximum iterations: 2,500
   7) converge on: gradient;
   8) convergence threshold: 0.05;
   9) conformational search method: torsional/low-mode sampling;
   10) torsional sampling options: intermediate;
   11) maximum number of steps: 1,000;
   12) energy window for saving structures: 21 kJ/mol;
   13) eliminate redundant conformers using maximum atom deviation cutoff: 0.5.
(2) Fine geometry optimization was performed with the Jaguar module based on the density functional theory (DFT) with the following parameters:
   1) theory: B3LYP-D3;
   2) charge: 1;
   3) reference: 6-31G**;
   4) spin multiplicity: 1;
   5) Hamiltonian: nonrelativistic;
   6) SCF accuracy level: quick;
   7) initial conjecture: atomic overlap;
   8) convergence criteria: 48 maximum iterations, 5e-05 energy change, and 5e-06 RMS density matrix change;
   9) convergence scheme: DIIS;
   10) solvent: H₂O;
   11) gas phase reference energy: optimized gas phase structure;
(3) MD simulations were performed to investigate major intermolecular interactions in self-assembled complexes by using the packing mode for the two Azo-C10-N+ molecules with acrylic acid via the Desmond module in Schrödinger Suites with the following parameters:
   1) force field: OPLS3e;
   2) solvent model: TIP4PD;
   3) ion placement: two bromide ions;
   4) boundary conditions: orthorhombic box shape, box size calculation method (buffer);
   5) simulation time: 20 ns, recording interval: 40 ps;
   6) approximate number of frames: 500;
   7) ensemble class: NPT;
   8) temperature: 300 K;
   9) pressure: 1.01325 bar;
   10) thermostat method: Nose-Hoover chain;
   11) coulombic interaction cutoff radius: 9.0 Å. Intermolecular interactions were analyzed at every 10 frames.

### 3. UV-Vis spectroscopy

In order to examine the absorption spectra of the isomeric states of the Azo-C10-N+ molecules, UV-Vis spectrometry (Agilent 8453) was performed at a wavelength in the range of 200 nm to 700 nm by treating the molecules alternately with UV light (365 nm, 0.25 W/cm², 30 sec) or Vis light (448 nm, 0.2 W/cm², 30 sec) .

The maximum absorption peaks at 315 nm and 345 nm corresponded to cis-Azo-C10-N+ and trans-Azo-C10-N+ of the self-assembled complex, respectively. The residual cispercentages of the Azo-C10-N+ molecules at various time points after quick UV treatment (365 nm, 0.25 W/cm², 30 sec) were determined using the relative absorption values at 375 nm at various time points. A lower absorption value at about 375 nm after UV treatment indicates a cis-percentage, whereas a higher absorption value at about 375 nm after Vis treatment indicates a trans-percentage. The UV-Vis absorption spectrum of β-carotene, a visible light-absorbing natural dye, was also measured.

### 4. Transmission electron microscopy (TEM)

The uniformities of the sizes and shapes of the core and core-shell of the upconversion nanoparticle were characterized by TEM imaging (Tecnai F20, FEI).

### 5. High resolution-transmission electron microscopy (HR-TEM) of upconversion nanoparticle (core-shell portion)

HR-TEM imaging (Tecnai F20, FEI) was performed at an operating voltage of 200 kV to identify the lattice patterns of the core and core-shell at atomic resolution. The average spacing between adjacent grids was labeled to identify a hexagonal crystalline phase, and the shell epitaxially grown on the core was identified.

### 6. High-angle annular dark field-scanning transmission electron microscopy (HAADF-STEM) of upconversion nanoparticle (core-shell portion)

In order to characterize the uniformities of the sizes and shapes of the core-shell nanostructures in the upconversion nanoparticles, HAADF-STEM imaging was performed using Talos 200x TEM (FEI Co.) with a spot size of 6 and a probe size of 2 nm at 200 kV. In the images, the shells of the upconversion nanoparticles appeared slightly darker than the cores.

### 7. Energy-dispersive X-ray spectroscopy (EDS) mapping

In order to confirm the uniform distribution of F and Y elements in the core and shell of the upconversion nanoparticle and the presence of Yb and Tm elements only in the core, EDS mapping was performed with the same settings used for HAADF-STEM.

### 8. Dynamic light scattering (DLS) analysis

DLS analysis (Zetasizer Nano ZS, Malvern, UK) was performed at room temperature to confirm the uniform diameter distribution of the cores and core-shells of the upconversion nanoparticles. As a result, it was confirmed that cores having a size of 34±0.9 nm grew into core-shells having a size of 47±1.2 nm.

### 9. Photoluminescence measurement

In order to confirm the efficiency of the upconversion nanoparticles coated with the natural dye β-carotene that absorbs visible light, the photoluminescence spectra were analyzed using a Hitachi F-7000 spectrophotometer that irradiates a 980 nm NIR diode laser. Each of the core, the core @ shell, and the core @ shell @ coating portion was measured. The photoluminescence intensity was normalized to the maximum peak intensity in the UV region at 340 nm. The ratio of UV to Vis photoluminescence was calculated by dividing the maximum peak intensity of UV light (340 nm) by that of the blue Vis region (450 nm). The UV peaks originated from the electronic transitions of ¹I₆ → ³F₄ and ¹D₂ → ³H₆ in the Tm³⁺ element, whereas the blue peaks originated from the electronic transitions of ¹D₂ → ³F₄ and ¹G₄ → ³H₆ in the Tm³⁺ element.

### 10. Scanning electron microscopy (SEM)

To confirm that the substrate surface was completely coated with the upconversion nanoparticles, SEM imaging (FEI, Quanta 250 FEG) was performed. Before SEM imaging, the substrate coated with the upconversion nanoparticles was completely dried and then coated with platinum for 90 seconds.

### 11. Fluorescence tracking of self-assembled complex by loading of fluorescent dye in process of forming self-assembled complex

In order to track the self-assembled complex through fluorescence, 3 mL of PBS containing ligand-bearing PAA (16.8 µmol) was mixed with 3 mL of PBS containing the Azo-C10-N+ molecule (3.42 µmol), and the sonicated for 5 minutes. Thereafter, a PBS solution containing 30 µL of 0.2 M redfluorescent rhodamine 6G dye was added thereto, and the mixture solution was further sonicated for 5 minutes to load the dye (rhodamine 6G) into the self-assembled complex. After centrifuging the solution at 1,000 rpm for 10 minutes, the supernatant in which unloaded rhodamine 6G remained was discarded. 1 mL of the self-assembled complex loaded with rhodamine 6G was attached onto the upconversion nanoparticle-coated substrate by treatment at room temperature for 30 minutes. The substrate was then imaged with a confocal microscope to visualize and quantify the number of the self-assembled complexes on the substrate.

### 12. Quantification of number of RGD ligands in self-assembled complex

The Nanodrop A280 method (NanoDrop 2000 Spectrophotometer, Thermofisher Scientific) was used to examine whether the RGD ligands contained in the self-assembled complex were saturated and maintained constant during cis/trans switching of the photoswitchable isomer. The self-assembled complex was synthesized in PBS, swollen by treatment with UV light (365 nm, 0.25 W/cm², irradiated for 1 minute), and deswollen by treatment with Vis light (448 nm, 0.2 W/cm², irradiated for 10 minutes). Thereafter, centrifugation was performed (at 1,000 rpm for 10 minutes), and the self-assembled complex was filtered using a syringe filter having a pore size of 1.2 µm.

The amount of the ligand-bearing PAA remaining unreacted in the supernatant was measured by tracking the tyrosine group of cyclo-RGDyK (cyclo-Arg-Gly-Asp-d-Tyr-Lys) bound to PAA. The amount of ligands unbound to the self-assembled complex was determined by subtracting the amount of unreacted ligand-bearing PAA in the supernatant from the amount of initially added ligand-bearing PAA. The measured number of ligands was divided by the calculated number of self-assembled complexes to determine the number of ligands per self-assembled complex.

### 13. Extent of swelling of self-assembled complex during switching

To examine the water absorption-mediated swelling of the self-assembled complex, the extent of swelling was calculated as the percentage of the wet weight, which was measured after UV treatment-mediated swelling (365 nm at 0.25 W/cm²) or Vis treatment-mediated deswelling (448 nm at 0.2 W/cm²) and divided by the dry weight of the self-assembled complex. 3 mL of PBS containing 1.5 mg of Azo-C10-N+ and 3 mL of PBS containing 30 mg of ligand-bearing PAA were mixed together and sonicated for 10 minutes to induce the formation of a self-assembled complex. The mixture was treated with UV or Vis light and centrifuged at 1,000 rpm for 10 minutes, and the supernatant was discarded to remove unreacted PAA, and then the wet weight of the self-assembled complex was measured. The wet self-assembled complex was dried in air for 7 days and the dry weight thereof was measured. The extent of swelling was calculated as the ratio of the dry weight to the wet weight of the self-assembled complex.

### 14. Real-time confocal microscopy imaging for fast and reversible swelling and deswelling of self-assembled complex on substrate

Real-time confocal microscopy imaging (K1 Fluo, Nanoscope systems) was performed to confirm the fast and reversible swelling and deswelling of the self-assembled complex on the substrate by real-time imaging. For clear imaging of the self-assembled complex, the self-assembled complex was attached to a glass substrate via hydrophilic interaction by incubating the substrate in a solution containing 400 µL of the self-assembled complex. Real-time imaging with video and time-lapse snapshot images was performed by swelling the self-assembled complex by irradiation with UV light (365 nm at 0.25 W/cm²) for 1 min and then deswelling the self-assembled complex by irradiation with Vis light (448 nm at 0.2 W/cm²) for 9 minutes. From the time-lapse snapshot images, it was confirmed that the self-assembled complexes were focused on the same plane, and lateral swelling and deswelling of the self-assembled complexes was confirmed.

These images were also used to quantify the number of self-assembled complexes per unit area (4992.7 ± 233.0 self-assemblies/mm²), and used to measure the diameter and lateral area of the self-assembled complex during swelling and deswelling on the substrate. The total side area (%) of the self-assembled complexes was calculated by multiplying the number of self-assembled complexes per unit area by the lateral area of each self-assembled complex.

### 15. Switching between swelling and deswelling of self-assembled complex via NIR and Vis light illumination for cell regulation

To test the effect of remotely regulating the swelling and deswelling of the self-assembled complex in the nanosystem by converting NIR light into UV light, experiments were conducted using macrophages. In the case of an experiment conducted on only the upconversion nanoparticle-coated substrate, the surface was passivated to prevent subsequent adhesion of the self-assembled complex for cellular regulatory effect. Macrophages (RAW 264.7, passage 5, ATCC) were plated on the nanosystem at a density of 5.3×10⁴ cells/cm² (cultured only at 0 h), and cultured in basal growth medium (DMEM, Dulbecco Modified Eagle Medium) supplemented with 10% heatinactivated fetal bovine serum and 50 U/ml penicillin and streptomycin antibiotics at 37°C under 5% CO₂. The substrate was irradiated alternately with NIR light (980 nm, 1 W/cm², irradiation for 1 min) and Vis light (448 nm, 0.2 W/cm², irradiation for 10 min) to induce swelling and deswelling of the self-assembled complex.

The effect of such remote control on cellular function (e.g., inflammatory/regenerative properties of macrophages) was examined either in inflammatory M1 polarization medium (containing 10 ng/mL of lipopolysaccharide) or in regenerative M2 polarization medium (containing each 20 ng/mL of interleukin (IL)-4 and IL-3). Correlation between the adhesion and inflammatory/regenerative functional properties of macrophages was examined using specific pharmacological inhibitors of ROCK (containing 50 µM Y27632 as an inhibitor), actin polymerization (containing 2 µg/mL cytochalasin D as an inhibitor), and myosin II (containing 10 µM blebbistatin as an inhibitor).

Experiments on negative controls were conducted using nanosystems without any one of the self-assembled complex/upconversion nanoparticle, the upconversion nanoparticle, the shell and coating portion of the upconversion nanoparticle, and the coating portion of the upconversion nanoparticle, and the self-assembled complex.

### 16. Confocal immunofluorescence imaging

In order to evaluate the effect of culturing cells on the nanosystem and remotely regulating the self-assembled complex by swelling and deswelling the self-assembled complex, confocal immunofluorescence imaging was performed.

Cultured macrophages were washed twice with D-PBS, fixed with 4% paraformaldehyde (PFA) for 10 minutes, and then washed four times with D-PBS. The fixed macrophages were blocked and permeabilized with blocking buffer (PBS containing 3% bovine serum albumin and 0.1% Triton-X 100) for 1 hour at 37°C, and then incubated with primary antibody for 16 hours at 4°C.

Thereafter, the macrophages were washed 4 times with PBS containing 0.5% (v/v) Tween 20, and then treated with a blocking buffer containing secondary antibody and phalloidin for 45 minutes at room temperature. Next, the macrophages were washed 4 times with PBS containing 0.5% (v/v) Tween 20, covered with DAPI antifade, and mounted on a glass slide.

Immunofluorescence-stained macrophages were imaged using a confocal microscope (LSM700, Carl Zeiss) with the same laser exposure and image acquisition settings for all groups, and then quantitatively analyzed using ImageJ software. The number of adhered cells was calculated using DAPI-positive cell nuclei from 5 different images, whereas the area of adheres cell area and the elongation factor (ratio of long axis to short axis) were calculated using F-actin-stained cells from 5 different images. Protein expression in the cells was calculated by quantifying the protein fluorescence intensity of F-actin-stained cells using five different images.

### 17. Western blotting analysis

Western blotting analysis was performed to quantify the effect of swelling and deswelling of the self-assembled complex on macrophages. Total protein was extracted from cultured macrophages by using 400 µL of a PRO-PREP^{™} protein extraction solution (iNtRON Biotechnology) containing 10 µL of a proteolytic inhibitor cocktail. The extracted protein concentration was quantified using a Thermo Scientific^{™} Pierce^{™} BCA Protein Assay Kit. The extracted proteins were denatured and separated by gel electrophoresis using polyacrylamide sodium dodecyl sulfate-gel (SDS-gel, 10%) at 110 V for 55 minutes, and then transferred to polyvinylidene fluoride (PVDF) membranes at 120 V for 90 min. The transferred proteins were washed with tris-buffered saline containing 0.1% Tween 20 (TBST) buffer for 10 min, and blocked with blocking buffer (TBST containing 5% skim milk) at 4°C overnight.

Thereafter, a blocking buffer containing primary antibodies against iNOS (135 kDa), Arg-1 (37 kDa) and GAPDH (36 kDa) was applied to the blocked proteins for 1 hour at room temperature. The proteins treated as described above were then washed three times with TBST buffer for 5 minutes each, and incubated with anti-HRP-conjugated secondary antibodies for 1 hour at room temperature. Next, the proteins were washed with TBST buffer and treated with ECL Western blotting reagent (Immobilon Western Chemiluminescent HRP Substrate, MERCK-Millipore). The proteins were then analyzed using a Linear Image Quant LAS 4000 chemiluminescent imaging system. The expression levels of the target proteins were presented after normalization to that of GAPDH.

### 18. Time-regulated remote control of dual-wavelength light-mediated protein delivery by self-assembled complex

To examine whether the self-assembled complex can be used as a protein delivery vehicle regulated by dual-wavelength light, 3 mL of PBS containing ligand-bearing PAA (16.8 µmol) was mixed with 3 mL of PBS containing the Azo-C10-B+ molecule (3.42 µmol), and then sonicated for 5 minutes. Thereafter, PBS containing 60 µL of recombinant murine IL-4 (Peprotech, catalog #: 214-14, 10 µg/mL) was added thereto, and then the mixture solution was vortexed for 5 min so that the IL-4 protein was loaded onto the self-assembled complex. The solution was then centrifuged at 1,000 rpm for 15 min, and the supernatant containing unloaded IL-4 was discarded. 40 mL of PBS was gently added to the protein-loaded self-assembled complex to be suspended, and then placed in a dialysis bag with a 100 kDa molecular weight cutoff. The protein-loaded self-assembled complex was irradiated with UV light (365 nm, 0.25 W/cm²) and Vis light (448 nm, 0.2 W/cm²) twice for 1 minute each at 30-min intervals. 400 µL of the supernatant that passed through the dialysis bag was collected at various time points, and centrifuged at 10,000 rpm for 15 minutes to remove unreacted self-assembled complexes. The supernatant was diluted appropriately, and the concentration of released IL-4 protein was quantified using ELISA (Abcam, catalog #: ab100710). Thereby, cumulative protein release profiles for time-regulated swelling and deswelling of the self-assembled complex were obtained.

### 19. Switching between swelling and deswelling of self-assembled complex in vivo by irradiation with NIR light and Vis light

As a proof-of-concept for confirming the effect of NIR and Vis light irradiation on host cell regulation *in vivo,* the nanosystem was implanted subcutaneously into mice. The experiment was conducted on two-month-old balb/c mice after obtaining approval from the Institutional Animal Care and Use Committee at Korea University. 44 2-month-old balb/c mice were anesthetized through an intraperitoneal injection of a mixture of 100 µL of alfaxan and rompun (2:1 (v/v)). In order to ensure constant delivery of protein (regenerative/antiinflammatory IL-4) to host cells to respond to the initial host response favoring inflammatory, PBS containing 1 mL of the nanosystem was incubated for 30 minutes at room temperature to load IL-4 protein. Then, the IL-4 protein-loaded nanosystem was implanted on the backs of the mice through an incision having a length of 1.8 cm, and the incision was sutured. At 0 and 12 hours post-implantation, the backs (toward the substrate) of the mice were repeatedly irradiated alternately with a NIR laser (980 nm, 1 W/cm², irradiation for 1 min) and a Vis laser (448 nm, 0.2 W/cm², irradiation for 10 min) to promote the swelling/deswelling of the self-assembled complex.

To confirm the *in vivo* stability of the self-assembled complex, a rhodamine 6G-encapsulated nanosystem was also implanted and analyzed by confocal imaging. Before implantation and 6 hours after implantation, a red morphology nanosystem was identified, which was quantified using ImageJ software. 24 hours after implantation, host macrophages attached to the nanosystem were analyzed by confocal immunofluorescence imaging and flow cytometry. Host cells expressing any one of pro-inflammatory iNOS and pro-regenerative Arg-1 markers were identified to be host macrophages. The *in vivo* local and systemic toxicity of the nanosystem was assessed by histological analysis.

### 20. Flow cytometry analysis for cell regulation in vivo

To quantitatively analyze the effect of (UV-free) NIR and Vis light irradiation on host cell regulation, flow cytometry was performed. The nanosystem recovered 24 hours after transplantation was gently washed with ice-cold PBS containing 10% FBS, and then centrifuged at 3,000 rpm for 5 minutes to collect host cells. The collected cells were resuspended in 4% PFA for 10 minutes, and then blocked with PBS containing 3% BSA for 1 hour in the dark. The blocked cells were treated with inflammatory (CD68) or regenerative (CD163) primary antibodies in PBS containing 3% BSA for 1 hour in the dark with appropriate isotype controls. Thereafter, the cells were centrifuged, washed with PBS, and then treated with secondary fluorescent antibodies in PBS containing 3% BSA for 30 min in the dark. The fluorescently labeled cells were washed with PBS containing 3% BSA and 1% sodium azide, and then analyzed using a FACS Calibur and quantified using BD CellQuest Pro software (BD Biosciences). The data were normalized to each isotype control and then analyzed using FlowJo software to generate a histogram of mean fluorescence intensity.

### 21. Local and systemic in vivo toxicity

The materials of the nanosystem include β-carotene dye and Tween 80 as well as upconversion nanoparticles under clinical trials, and an experiment was conducted on whether these materials showed potential toxicity *in vivo* upon (UV-free) NIR and Vis light irradiation.

First, the nanosystem was implanted subcutaneously into mice. Histological analysis was performed to examine the cell structure before and 7 days after implantation. Subcutaneous tissue was analyzed to assess local toxicity *in vivo,* whereas various organs (e.g., liver, kidney, spleen and heart) were analyzed to assess systemic toxicity *in vivo.* Various tissues and organs were fixed with 4% PFA for 48 hours. The fixed tissue samples were then washed with DI water, dehydrated in ethanol-DI water mixtures with an ethanol concentration gradient (70%, 80%, 90%, 95% and 100% ethanol in that order) for 1 hour (2 times each ethanol concentration), and then embedded in paraffin at 60°C overnight. The paraffin-embedded sample was sectioned at 5 µm thickness using a microtome (HistoCore Multicut, Leica RM2125 RTS Biosystem), and the sections were mounted on a glass slide, deparaffinized using a xylene substitute, and then rehydrated in DI water-ethanol mixtures with an ethanol concentration gradient (70%, 80%, 90%, 95% and 100% ethanol in that order). The rehydrated sections were rehydrated with a hematoxylin and eosin (H&E) solution and mounted on glass slides to examine changes occurring in various tissues and the cellular tissues of organs before and 7 days after NIR or Vis light irradiation after implantation of the nanosystem.

### 22. Statistical analysis

All of the experiments were repeated independently twice or three times. Graphpad Prism 8 software was exploited to perform all statistical treatments. Statistically significant differences between groups were set at p values < 0.05. Two-tailed Student's t-tests were performed to analyze differences between two different groups while one-way analysis of variance (ANOVA) along with Tukey-Kramer post-hoc tests was exploited to compare multiple groups.

In the following experiments and the drawings, NIR, Vis (or Vis light) and UV means that irradiation with the corresponding light was done. That is, for example, NIR-Vis means that NIR light irradiation and then Vis light irradiation were done.

The core in the upconversion nanoparticle means a structure having only a core without a shell and a coating portion. In addition, the core-shell, core-shell portion, core@shell, or core@shell portion in the upconversion nanoparticle refers to a structure having only a core and a shell covering the entire surface of the core without a coating portion. In addition, if there is no restriction on the components in the upconversion nanoparticle, core@shell@dye refers to an upconversion nanoparticle having a structure in which the coating portion completely covers the surface of the core-shell.

### [Experimental Example 1] Characterization of Nanosystems

### 1. Characterization of photoswitchable isomer

As a photoswitchable isomer that mediate the selfassembly of self-assembling complexes, an Azo-C10-N+ molecule was synthesized.

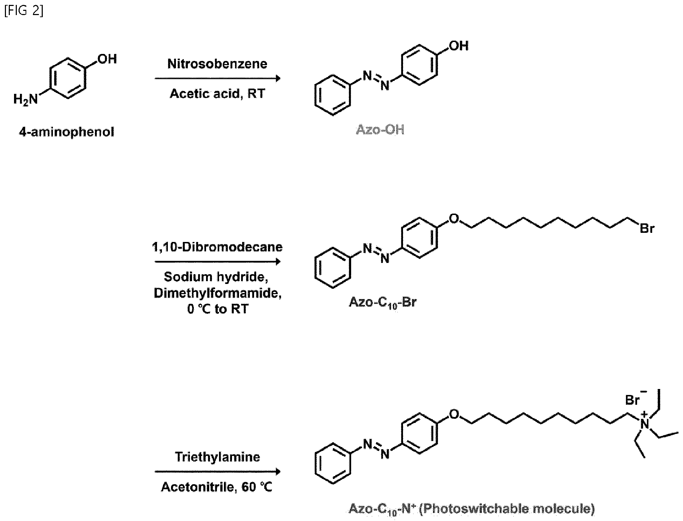

FIG. 2 shows a scheme for synthesis of the Azo-C10-N+ molecule. First, Azo-OH was synthesized and then converted into Azo-C10-Br containing a hydrophobic tail spacer between the aromatic group and Br. Thereafter, Br in the Azo-C10-Br molecule was replaced with N+ to obtain Azo-C10-N+ molecules that could be stacked in a self-assembled complex through π-cation interaction and π-π interaction.

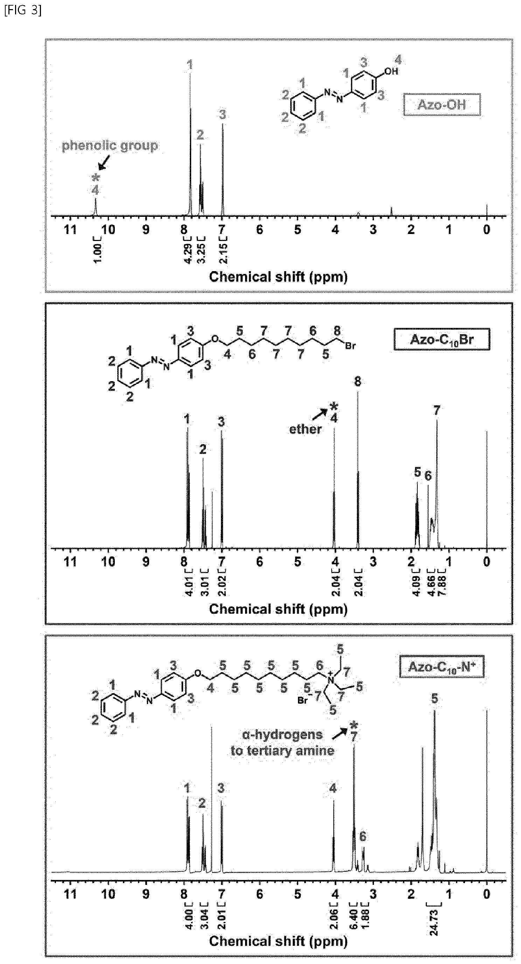

FIG. 3 shows ¹H NMR spectra indicating the synthesis of the molecules. ¹H NMR spectra of the Azo-OH, Azo-C10-Br and Azo-C10-N+ molecules show characteristic peaks corresponding to a phenol group (10.34 ppm), an ether group (4.05 ppm) and quaternary amine α-hydrogen (3.54-3.25 ppm), and shows that the photoswitchable Azo-C10-N+ molecule was successfully synthesized.

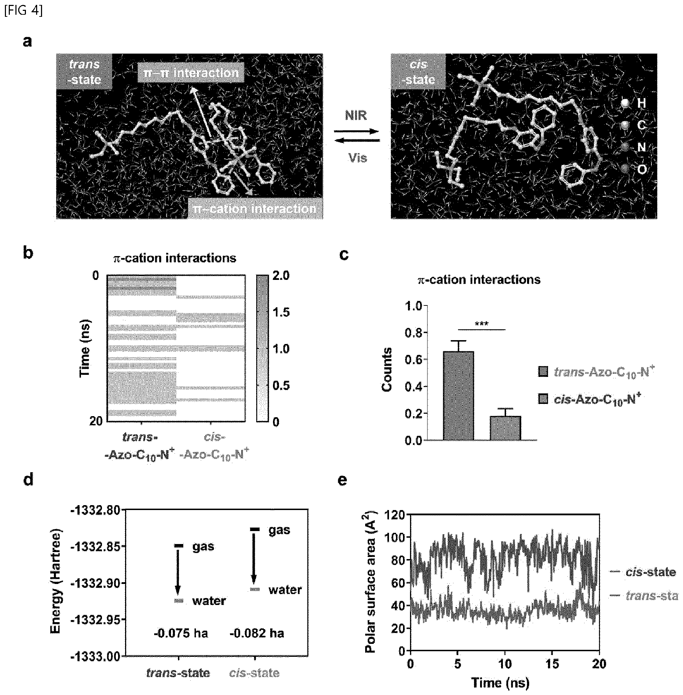

FIG. 4 shows the results of molecular dynamics simulation (MD) analysis of the Azo-C10-N+ molecule.

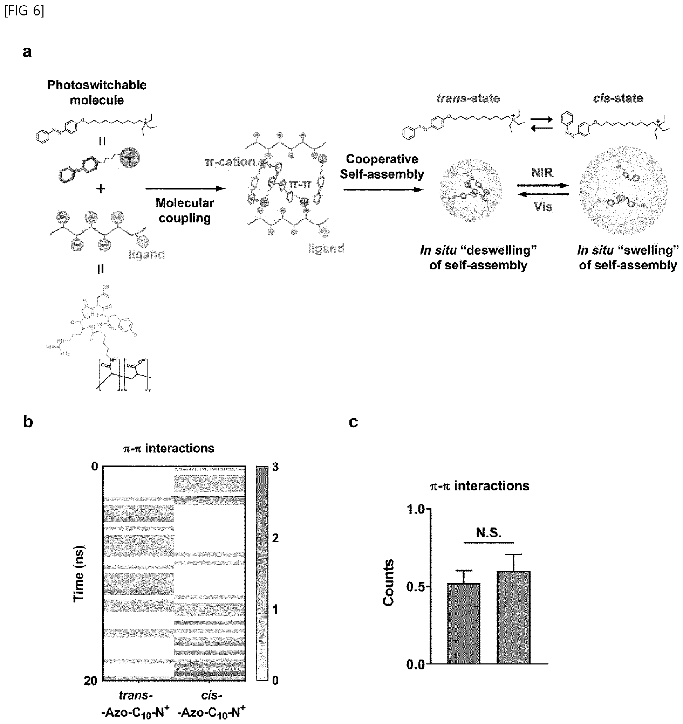

Molecular dynamics simulations show that switching between the trans- and cis- states of the Azo-C10-N+ molecule regulate intramolecular stacking and water absorption. Snapshots of MD simulations for the Azo-C10-N+ molecules in the trans- and cis-states show that intramolecular π interactions in the Azo-C10-N+ molecule are promoted in the trans-state (FIG. 4a). This is because the nitrogen cation and the hydrophobic tail spacer mediate the stacking of the Azo-C10-N+ molecules. The heat map chart and frequency number of π-cation interactions in the Azo-C10-N+ molecules at 20 ns of MD simulation further confirm that these interactions are highly promoted in the trans state than in the cis state. On the other hand, the degree of π-π interactions is similar between the cis- and trans-states. Such characteristics can be confirmed through FIGS. 6b and 6c and FIGS. 4b and 4c.

FIGS. 4D and 4E show the values obtained by density functional theory (DFT) calculation for each isomer of the Azo-C10-N+ molecule. The Azo-C10-N+ molecule in the cis state shows higher hydrogenation energy and a greater polar surface area, indicating that the affinity for water in the cis-state is much greater than that in the trans-state.

Taken together, the twisted structure of the cis-Azo-C10-N+ molecule with inhibited π-cation interaction improves the polarity and hydrophilicity of the molecule, and thus promotes swelling of the self-assembled complex by absorbing water. In contrast, the π-cation interaction in the relatively less polar trans-Azo-C10-N+ molecule inhibits water absorption into the self-assembled complex, making the self-assembled complex dense.

### 2. Characterization of ligand-bearing polymer and self-assembled complex

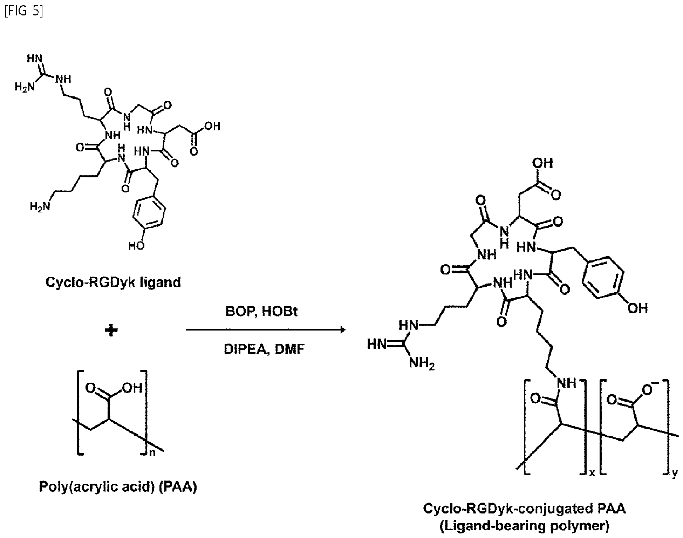

In order to introduce the RGD ligand into the self-assembled complex, a ligand-bearing and negatively charged polymer was synthesized, and stacked Azo-C10-N+ molecules were bound thereto by electrostatic interaction. In this case, as the polymer, polyacrylic acid (PAA) was used, and as the RGD ligand, a cyclo-RGDyK with a lysine residue bearing an amine group was used. FIG. 5 shows the process of conjugating the ligand to the carboxylate group of PAA.

The self-assembled complex can be formed by the aggregation of ligand-bearing PAA while facilitating the stacking of Azo-C10-N+ molecules by π-cation interaction and π-π interaction. FIG. 6a schematically shows the process of forming this self-assembled complex.

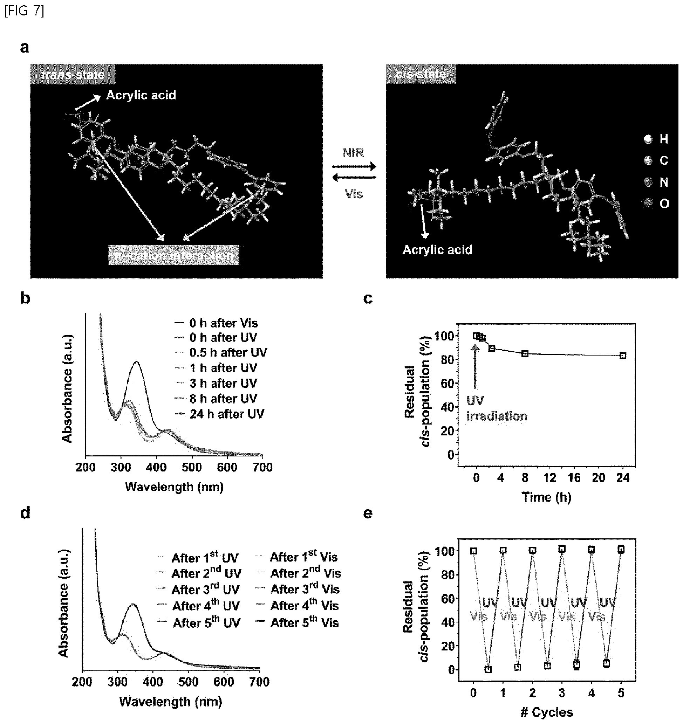

MD simulations using the trans and cis forms of the Azo-C10-N+ molecule of the self-assembled complex were performed, and the results are shown in FIG. 7.

FIG. 7a shows that the Azo-C10-N+ molecules in the trans and cis states remain linked with acrylic acid.

To assess the stability (e.g., lifetime) of the cis-Azo-C10-N+ molecule in the self-assembled complex, the self-assembled complex was irradiated with UV light to promote the cis state, and then the time-resolved conversion from the cis state to the trans state was examined. To this end, an experiment was performed using UV-Vis spectroscopy and the results are shown in FIGS. 7b to 7e.

The results of UV-Vis spectroscopy showed that the maximum peak intensity after visible light irradiation for formation of the trans state appeared at 345 nm, and appeared at 315 nm after UV light irradiation at 0 hours for formation of the cis state. From these results, it can be confirmed that almost 80% or more of the cis state remained at 24 hours after UV irradiation for 30 seconds, indicating the stability of the cis state on a day-scale (FIGS. 7b and 7c).

This long lifetime of the cis state corresponds to the lifetime of an azobenzene derivative designed to exhibit a blue-shifted absorption peak in the trans state. UV-Vis absorption spectra obtained by performing 5 cycles or more, each consisting of UV irradiation for 30 seconds followed by Vis irradiation for 30 seconds, and time-dependent residual cis population, confirm that fast and reversible conversion between the cis and trans populations during 5 cycles is possible (FIGS. 7d and 7e).

### 3. Characterization of upconversion nanoparticles

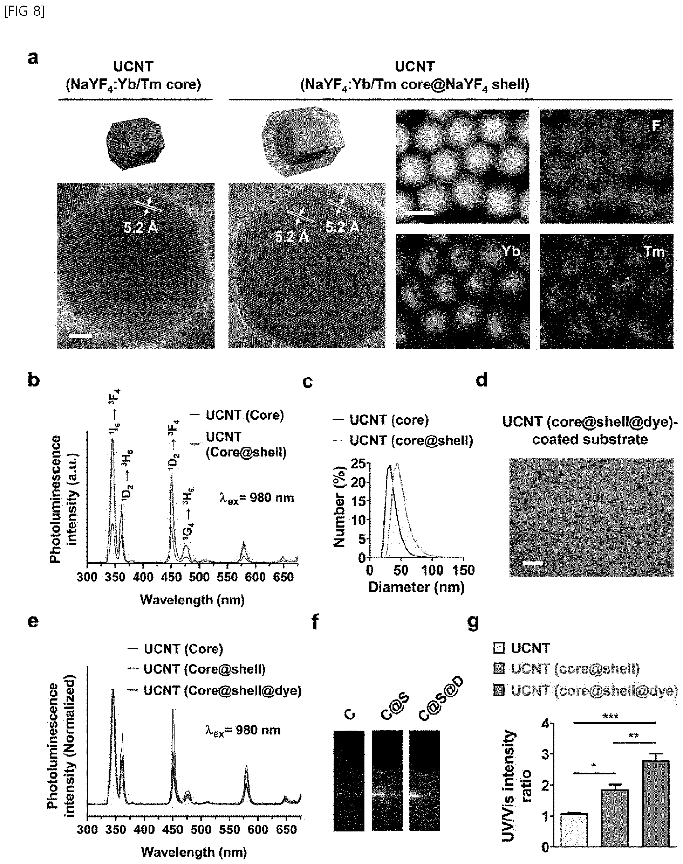

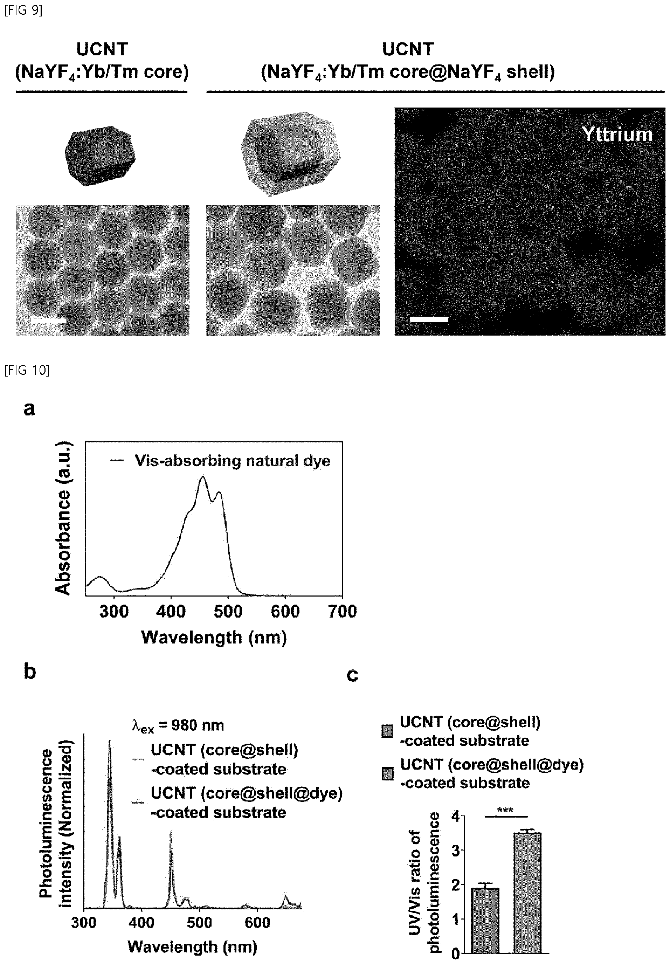

In order to remotely control the reversible swelling and deswelling of the self-assembled complex by UV-free (and thus tissue-penetrating) stimulation, upconversion nanoparticles that upconvert NIR to UV light were synthesized. The upconversion nanoparticles were designed to have a core@shell structure (NaYF₄:Yb,Tm@NaYF₄) by epitaxially growing the shell on the core to prevent surface defects. The results of analysis of the upconversion nanoparticles are shown in FIGS. 8 and 9.

The HR-TEM images in FIGS. 8a and 9 show that the synthesized core and shell had a uniform size distribution and a uniform shape. The images also show that both the core (NaYF₄:Yb,Tm) and the shell (NaYF₄) had a defect-free hexagonal crystal structure. The HAADF-STEM images and EDS mapping in these figures show that not only F and Y elements were uniformly distributed in the core and shell structures, but also Yb and Tm dopant elements were uniformly present only in the core.

FIG. 8b shows that the core of the synthesized upconversion nanoparticles exhibited photoluminescence peaks in the UV and Vis (blue) regions below NIR (980 nm) excitation. In addition, from FIGS. 8b and 8c, it can be confirmed that, when the shell of the upconversion nanoparticle was epitaxially grown on the core, the fluorescence intensity and diameter of the core-shell structure significantly increased compared to when only the core was present.

FIG. 10 shows the results of analyzing photoluminescence depending on the presence or absence of the coating portion in the upconversion nanoparticle. In order to selectively induce the formation of cis-Azo-C10-N+ mediating the swelling of the self-assembled complex by NIR-converted-UV light, NIR-to-UV or -Vis conversion was maximized using a natural dye. The natural dye used herein is β-carotene that absorbs visible light in the blue region. On the other hand, the formation of trans-Azo-C10-N+ can be induced by irradiating the self-assembled complex with visible light, and at this time, the self-assembled complex shrinks.

Thus, NIR needs to be converted to UV light beyond Vis to selectively induce cis-Azo-C10-N+. Eventually, upconversion nanoparticles may be obtained by coating the core-shell with a visible light-absorbing dye to form cis-Azo-C10-N+ by NIR irradiation. FIG. 8d shows that a substrate is coated with the upconversion nanoparticles formed as described above. The photoluminescence spectrum excited by NIR (980 nm) was normalized to the peak in the UV region of 340 nm, and the peak intensity at 450 nm was the highest in the core and gradually decreased toward the core-shell and the upconversion nanoparticles formed up to the coating portion (FIG. 8e). From FIG. 8f showing photoluminescence excited by NIR, it can be confirmed that blue photoluminescence appeared very strongly in the core-shell, but the intensity decreased toward the upconversion nanoparticles formed up to the coating portion.

FIG. 8g shows the results of quantifying the UV (340 nm)/Vis (450 nm) photoluminescence intensity ratio, and shows that NIR-to-UV upconversion in the upconversion nanoparticles is more favorable than NIR-to-Vis light upconversion.

FIGS. 8d and 10b and 10c show that the upconversion nanoparticles completely covered the surface of the substrate with a uniform distribution through hydrophilic interaction, and exhibited a very high UV/Vis intensity ratio.

Therefore, these results confirm that the upconversion nanoparticles selectively upconvert NIR to UV, and the intensity thereof is also high.

### 4. Characterization of nanosystem

A nanosystem was formed by conjugating the self-assembled complex to the upconversion nanoparticle-coated substrate by hydrophilic interaction. When this nanosystem is irradiated with NIR light, the NIR light is upconverted to UV light by the upconversion nanoparticles, and the UV light stimulates the self-assembled complex to convert the photoswitchable isomer (Azo-C10-N+) of the self-assembled complex to the cis-isomer, thereby swelling the self-assembled complex. In addition, when the nanosystem is irradiated with Vis light, the photoswitchable isomer of the self-assembled complex is isomerized to the trans-isomer, which deswells the self-assembled complex.

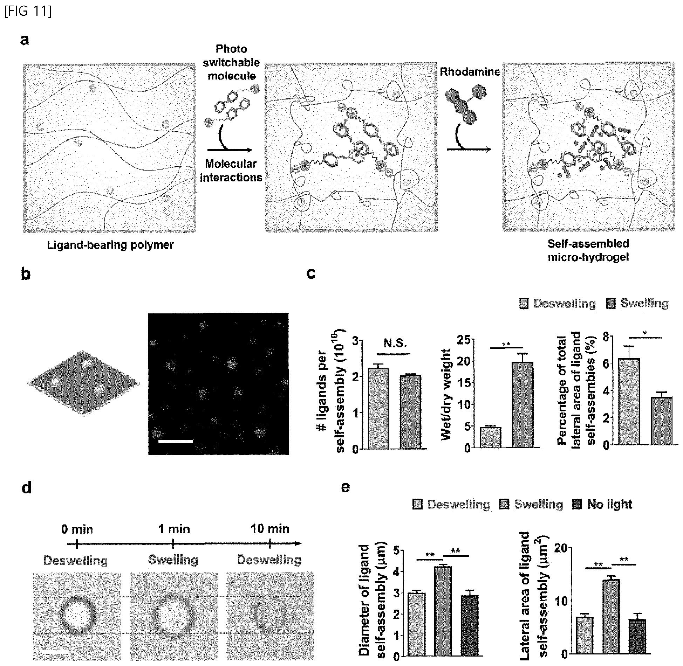

In order to examine the behavior of this nanosystem, the self-assembled complex was loaded with rhodamine and subjected to a swelling/deswelling experiment, and the results of the experiment are shown in FIG. 11.

FIG. 11a schematically illustrates a process in which rhodamine is loaded into the self-assembled complex. FIG. 11b shows a fluorescence image of the self-assembled complex, and it can be confirmed that strong red fluorescence was displayed by rhodamine loaded in the self-assembled complex.

FIG. 11c depicts graphs showing the results of quantifying various values resulting from swelling and deswelling of the self-assembled complex. The number of ligands per self-assembled complex was measured after swelling and deswelling of the self-assembled complex by dual-wavelength light, and was almost similar between the swelling state (2.23 ± 0.11 × 10¹⁰) and the deswelling state (2.03 ± 0.03 × 10¹⁰).

This means that the number of ligands exposed is more important than the number of ligands saturated. In addition, the value obtained by dividing the saturated wet weight of the self-assembled complex in the light-mediated swelling state by the dry weight greatly increased by 308% compared to that in the deswelling state, because the self-assembled complex in the swelling state absorbed water. Swelling of the self-assembled complex prevents twisting of the ligand-bearing polymer by reducing the stack structures of the Azo-C10-N+ molecules, and absorbs water into the swollen self-assembled complex. At this time, the ligands conjugated to the polymer are very exposed to the outside. In contrast, deswelling of the self-assembled complex increases the stack structures of the Azo-C10-N+ molecules, making the internal structure of the self-assembled complex dense, also increases the twisting of the polymer. Thus, in this case, water cannot be absorbed into the self-assembled complex, and only some of the ligands conjugated are exposed to the outside.

FIG. 11d depicts snapshots of real-time confocal microscopy images of the self-assembled complex, and shows the results of observing changes in the self-assembled complex after swelling for 1 minute and then deswelling for 9 minutes. From FIG. 11d, it can be confirmed that the axial changes resulting from swelling and deswelling of the self-assembled complex were minimized, whereas lateral swelling and deswelling occurred. This is due to a reversible hydrophilic interaction between the self-assembled complex and the substrate surface.

Summarizing FIGS. 11c to 11e for swelling and deswelling of the self-assembled composite, the diameter and the lateral cross-sectional area of the self-assembled complex and the percentage of the total lateral cross-sectional area of the self-assembled complexes reversely changed with very large differences depending on swelling and deswelling of the self-assembled complex. The percentage of the total lateral cross-sectional area of the self-assembled complexes means that cell adhesion can be sensitively and temporally regulated by swelling and deswelling of the self-assembled complex.

### [Experimental Example 2] Experiment on regulation of macrophage adhesion by nanosystem

An experiment was conducted on whether cell adhesion and polarization could be regulated using the swelling and deswelling properties of the nanosystem. To this end, the ligand conjugated to the polymer was used as a cell-adhering RGD ligand (Arg-Gly-Asp). As for the cell type, macrophages having a size similar to the size (3 to 5 µm) of the self-assembled complex were used to examine the direct correlation between the cell and the self-assembled complex.

Macrophages were placed on the nanosystem and irradiated with NIR light (980 nm, 1 W/cm², irradiation for 1 minute) and Vis light (448 nm, 0.2 W/cm², irradiation for 10 minutes), and the irradiated NIR light was upconverted into UV light.

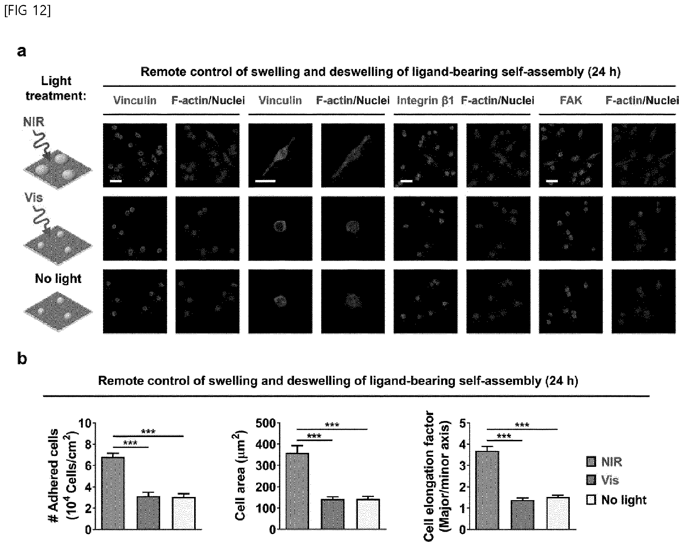

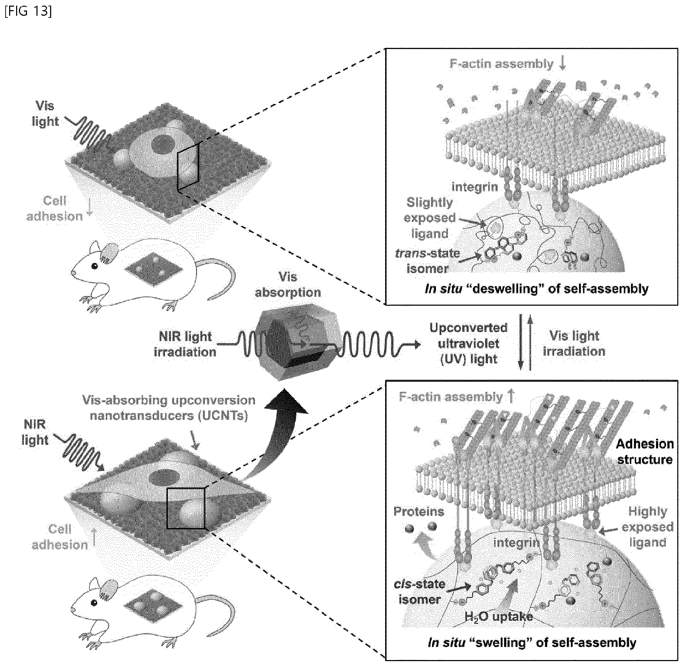

FIGS. 12 and 13 show the results of an experiment conducted on the effect of the nanosystem on macrophages, and schematically show the process of adhesion of macrophages by the nanosystem.

Through confocal immunofluorescence images of the NIR group in FIGS. 12a and 12b, it can be confirmed that the ligand was highly exposed at high adherent cell number, cell area and elongation factor, and the expression of integrin β1, F-actin filaments, and focal adhesion complexes increased, which means that macrophages easily adhere to the swollen self-assembled complexes.

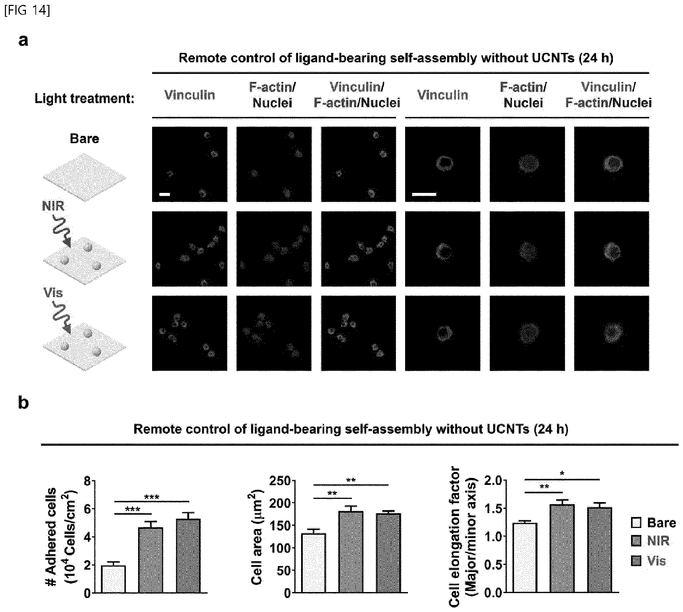

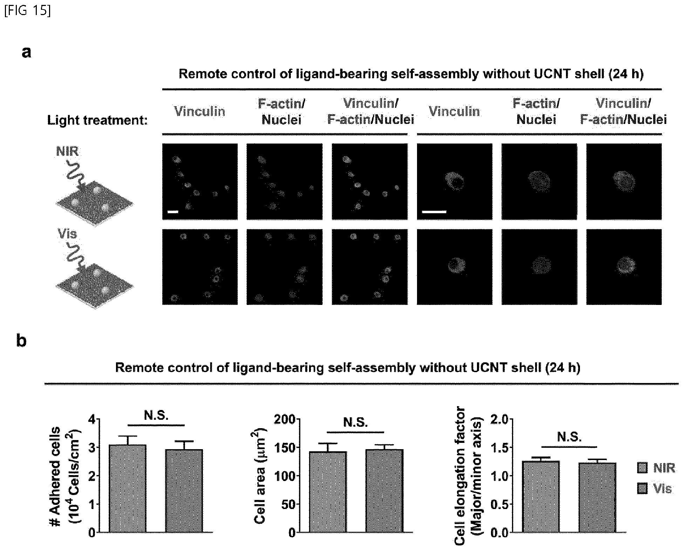

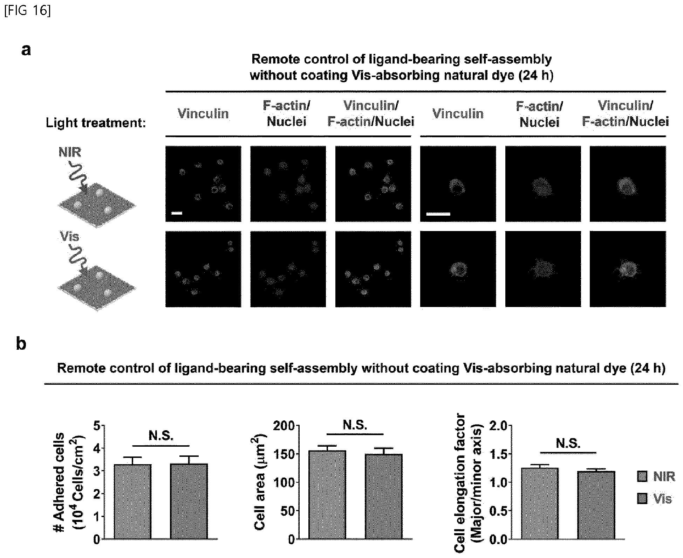

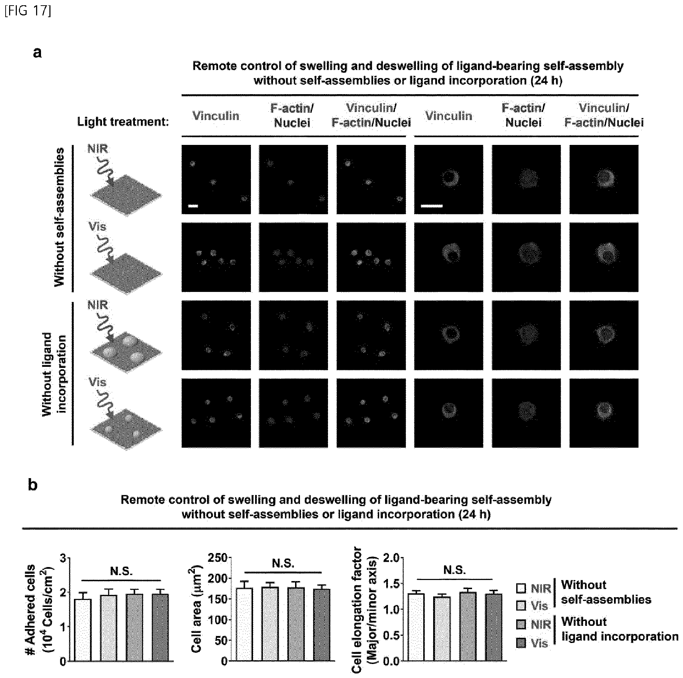

FIGS. 14 to 17 are experimental results for negative controls which respectively correspond to the case where there was no upconversion nanoparticle in the nanosystem and there was only the substrate, the case wherein the upconversion nanoparticle was formed only of the core, the case where the upconversion nanoparticle had only the core-shell portion, and the case where there was no self-assembled complex or ligand.

It can be confirmed that, in each negative control, there were no significant changes in the number of adhered cells, the area of adhered cells and the cell elongation factor, even though each negative control was irradiated with NIR or Vis light. This means that each component of the upconversion nanoparticle and the ligand in the nanosystem are essential for cell adhesion and regulation.

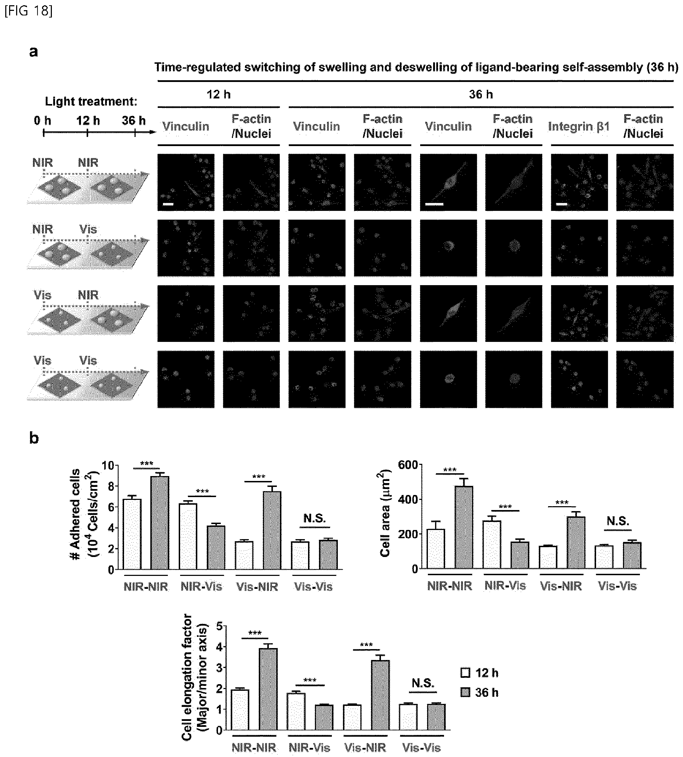

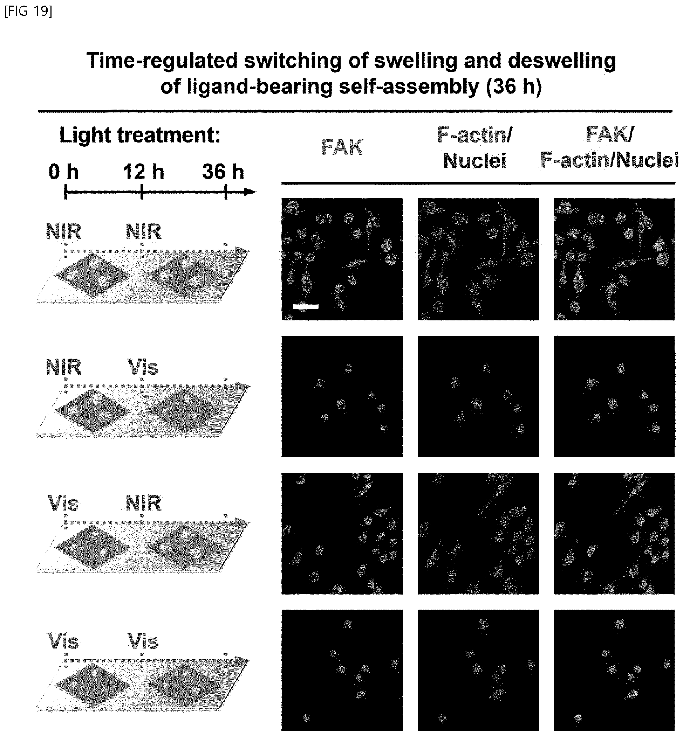

FIGS. 18 and 19 show the results of an experiment conducted to examine the effect of the nanosystem on cell adhesion after the nanosystem was irradiated alternately with NIR and Vis light.

The nanosystem was irradiated with NIR or Vis light at 0 hours and 12 hours, and cell adhesion was checked at 12 hours and 36 hours. Experimental groups were divided into "NIR-NIR", "NIR-Vis", "Vis-NIR", and "Vis-Vis" groups based on the irradiation method.

Interestingly, in the "NIR-Vis" group, cell adhesion was promoted due to NIR-mediated swelling at 12 hours, and cell adhesion was reversibly inhibited due to Vis-mediated deswelling at 36 hours.

This suggests that the photoswitchable nanosystem of the present invention could efficiently achieve time-regulated cell adhesion due to the long-term (24 hours) stability of Vis light-induced cis-Azo-C10-N+ molecules in the self-assembled complexes (see FIGS. 7b and 7c).

These results mean that the adhesion of macrophages can be regulated by irradiating the nanosystem with NIR or Vis light.

### [Experimental Example 3] Experiment on macrophage polarization by photoswitching of nanosystem

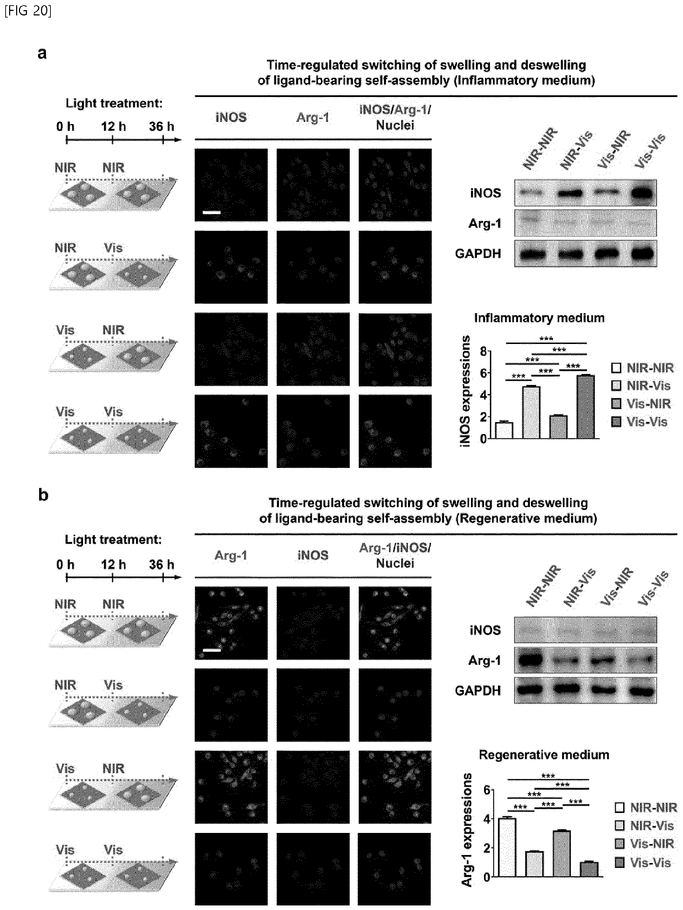

Next, an experiment was conducted on whether the nanosystem could be used to regulate macrophage adhesion and then macrophage polarization. The results of the experiment are shown in FIGS. 20 to 23.

In macrophages, it is known that strong cell adhesion complexes in an elongated form, including a distinct assembly of cytoskeletal F-actin filaments, activate rho-associated protein kinase (ROCK) that stimulates to have pro-regenerative/anti-inflammatory functions.

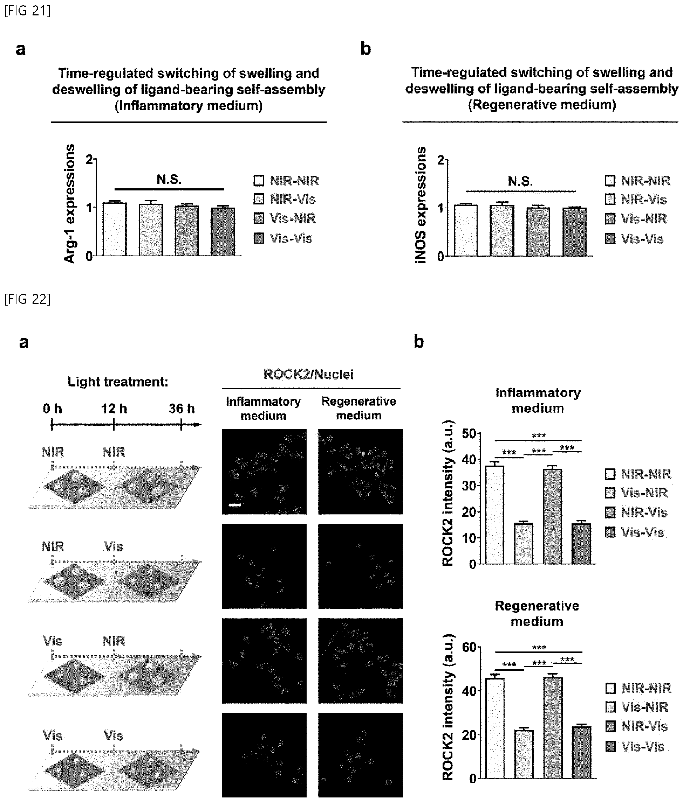

Confocal immunofluorescence images and Western blotting images showed that the groups ("NIR-NIR" and "Vis-NIR" groups) irradiated with NIR light later exhibited pro-regenerative/anti-inflammatory functions in each medium condition, and conversely, the groups ("NIR-Vis" and "Vis-Vis" group) irradiated with Vis light later exhibited high pro-inflammatory function (FIGS. 20 and 21).

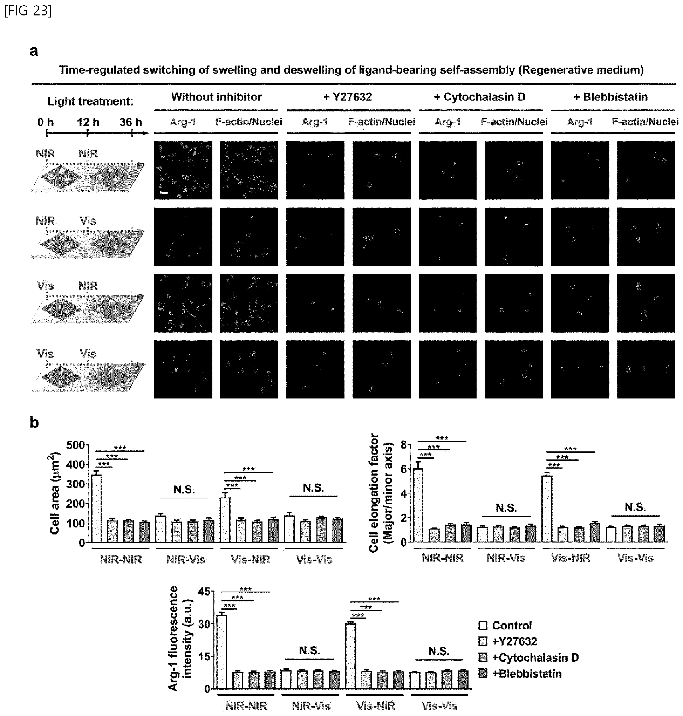

In addition, when an experiment was conducted in the presence of an inhibitor of each protein that interferes with cell adhesion, it can be confirmed that the pro-regenerative/anti-inflammatory functions were impaired, indicating that that there is a correlation between cell adhesion and cellular function (FIGS. 22 and 23).

In this experiment, the proteins were ROCK, actin polymerization, and myosin II, and inhibitors of these proteins were Y27632, cytochalasin D and blebbistatin, respectively.

These results suggest that, when the nanosystem is irradiated with NIR or Vis light, it may regulate not only macrophage adhesion but also macrophage polarization.

### [Experimental Example 4] Experiment on protein delivery by photoswitching of nanosystem

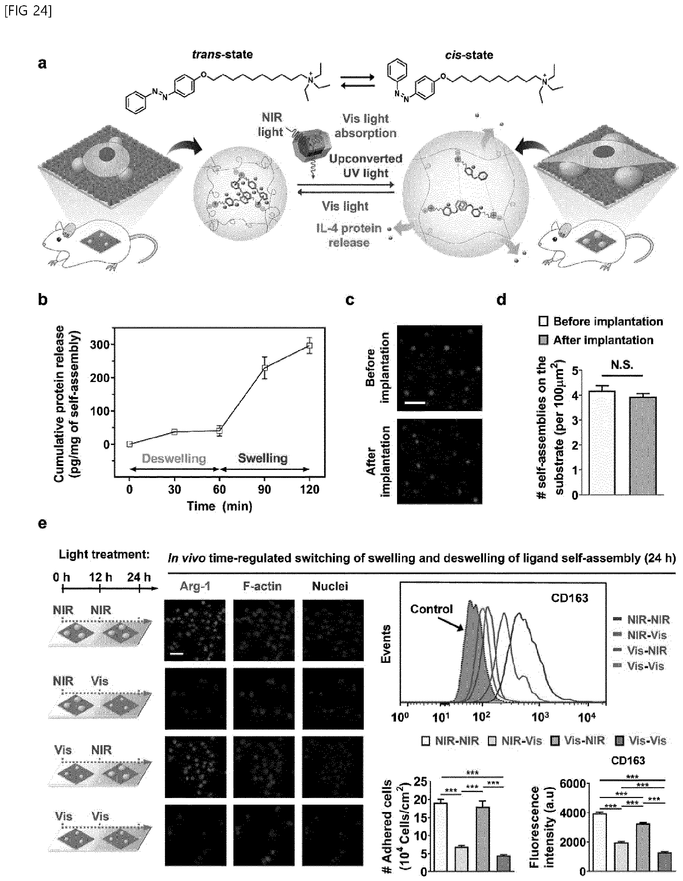

An experiment was conducted on whether substance delivery using the nanosystem could be achieved by irradiating the nanosystem with NIR or Vis light to control the swelling or deswelling time of the self-assembled complex. The results of the experiment are shown in FIG. 24.

FIG. 24a schematically shows a process in which Azo-C10-N+, which is a photoswitchable isomer, undergoes cis-trans isomerization by NIR or Vis light irradiation and a substance is delivered. The Azo-C10-N+ molecule is entangled with the ligand-bearing PAA by intramolecular interactions or untangled depending on the cis or trans conformation thereof. Here, interleukin-4 (IL-4) protein was used as the delivery substance and was loaded into the self-assembled complex.

Referring to FIG. 24b, it can be confirmed that, when the self-assembled complex was deswollen by irradiating the nanosystem with Vis light at 0 min and 30 min, the loaded protein was maintained in the self-assembled complex for 60 minutes. However, it can be confirmed that, when the self-assembled complex was swollen by irradiating the nanosystem with UV (UV upconverted from NIR) for 1 min at 60 and 90 min, the loaded protein diffused over time from 60 minutes and came out of the self-assembled complex.

Therefore, when the nanosystem is irradiated with NIR or Vis light, the substance loaded in the self-assembled complex can be delivered to a specific site, and the delivery time thereof can also be regulated.

### [Experimental Example 5] In vivo experiment on nanosystem

An experiment on macrophages and substance delivery was conducted by implanting the nanosystem into a living body.

In the course of the experiment, it could be confirmed that the behavior of the nanosystem mimicked tissues and organs such as lung, heart and brain, and dynamically changed the structure of the biopolymer network by repeated swelling and deswelling of the self-assembled complex through the movement of liquid. Host macrophages were analyzed in this experiment because of their important role in regulating late responses such as fibrosis and tissue regeneration by regulating the early inflammatory response.

First, the pro-regenerative IL-4 protein-loaded nanosystem was subcutaneously implanted into mice, and then whether the light stability of the self-assembled complexes in vitro was the same as that *in vivo* was examined. The results are shown in FIGS. 24c and 24d. From both the immunofluorescence images and the graph showing the results of quantifying the images, it can be confirmed that the number of implanted self-assembled complexes hardly changed.

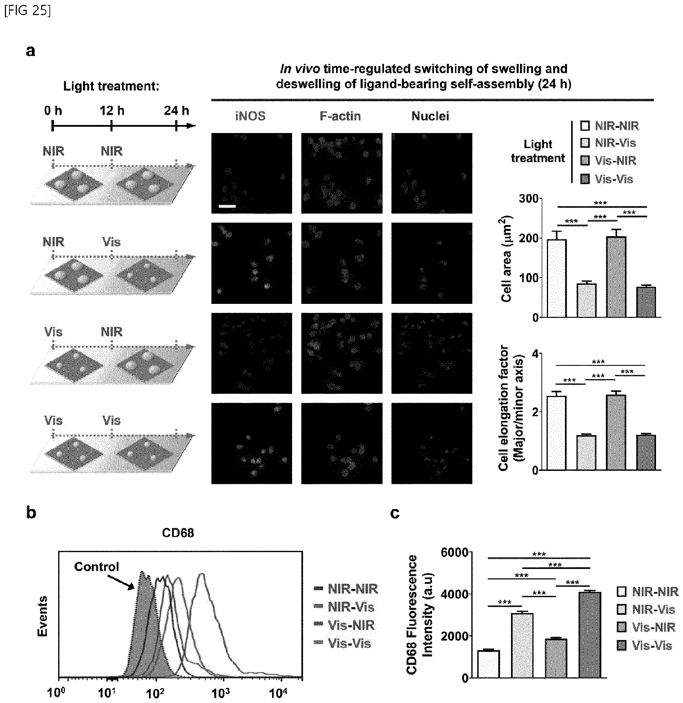

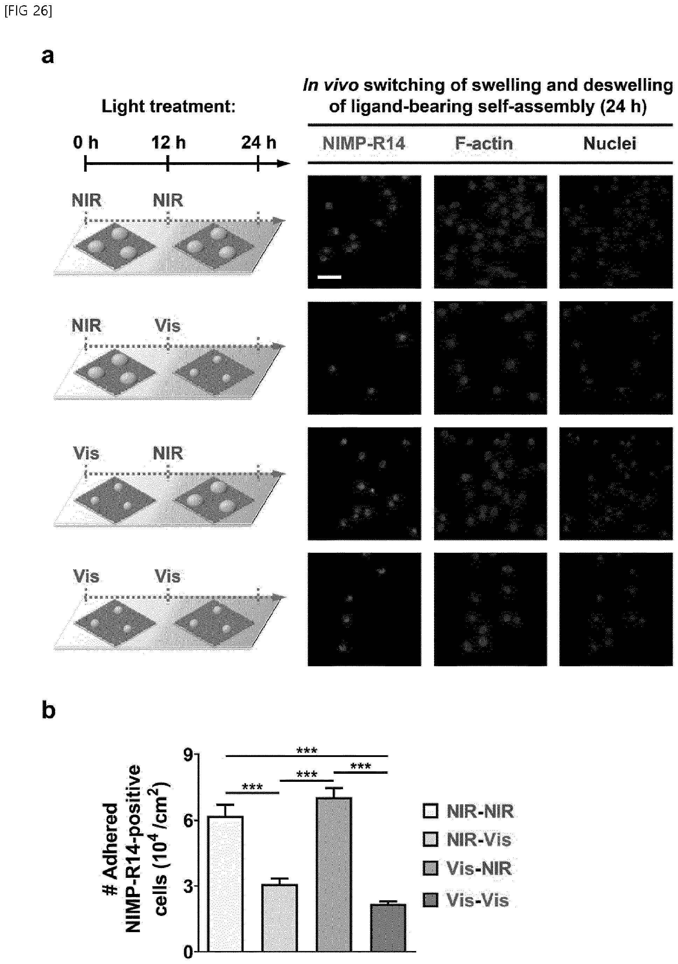

In addition, confocal immunofluorescence and flow cytometry were performed in the "NIR-NIR" and "Vis-NIR" groups, designed to exhibit swelling-mediated protein release and exhibit high ligand exposure in late responses, and the results are shown in FIGS. 24e, 25 and 26. It can be confirmed that, in the "NIR-NIR" and "Vis-NIR" groups, host macrophage adhesion and pro-regenerative/anti-inflammatory functions together with neutrophil recruitment were synergistically promoted compared to those in the "Vis-NIR" and "Vis-Vis" groups.

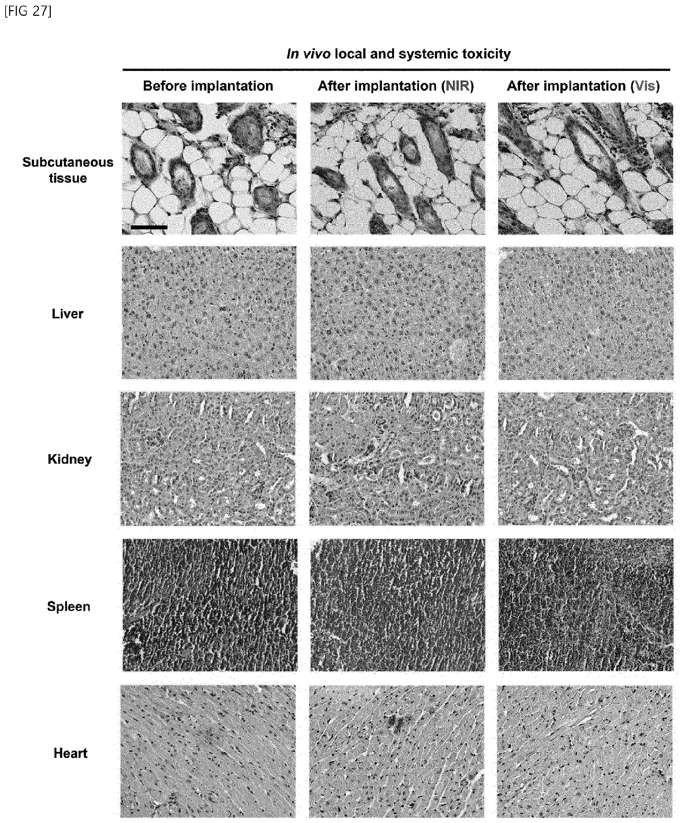

FIG. 27 shows the results of testing whether the nanosystem was not toxic *in vivo* when irradiated with NIR or Vis light. The non-toxicity test was performed for 7 days on local subcutaneous tissue and whole organs such as liver, kidney, spleen, and heart. FIG. 27 shows images of each tissue or organ before nanosystem implantation, 7 days after implantation, and 7 days after light irradiation, and no specific change in the cellular structure was found.

The nanosystem is a system that can be reversibly controlled without UV light, which means that macrophages and substance delivery can be regulated by irradiating NIR and Vis light without using UV light harmful to the living body.

According to the present invention, it is possible to provide a technology of collecting a biological sample, delivering a substance into a living body, and regulating macrophage adhesion and polarization, by controlling the self-assembled complex and the nanosystem including the same by irradiation with light.

While the present invention has been described with reference to the particular illustrative embodiments, it will be understood by those skilled in the art to which the present invention pertains that the present invention may be embodied in other specific forms without departing from the technical spirit or essential characteristics of the present invention. Therefore, the embodiments described above are considered to be illustrative in all respects and not restrictive. Furthermore, the scope of the present invention is defined by the appended claims rather than the detailed description, and it should be understood that all modifications or variations derived from the meanings and scope of the present invention and equivalents thereto are included in the scope of the present invention.

## Claims

1. A light-controllable self-assembled complex comprising:
a polymer complex comprising a ligand and a negatively charged polymer; and
a positively charged photoswitchable isomer bound to the negatively charged polymer complex,
wherein the photoswitchable isomer is present as a cis- or trans-isomer and undergoes reversible cis-trans isomerization in response to light, and
a volume of the light-controllable self-assembled complex reversibly swells or shrinks due to the isomerization of the photoswitchable isomer.

2. The light-controllable self-assembled complex of claim 1, wherein
the self-assembled complex reversibly swells and deswells in response to light in an environment with a aqueous liquid,
the self-assembled complex is present in a hydrogel form in the environment with the aqueous liquid, and
the self-assembled complex absorbs the aqueous liquid during swelling and releases the aqueous liquid during deswelling.

3. The light-controllable self-assembled complex of claim 2, wherein
the aqueous liquid absorbed into the self-assembled complex is collected and analyzed, and
the aqueous liquid comprises at least one of blood, plasma, serum, urine, saliva, cerebrospinal fluid, tears, sweat, feces, ascites, amniotic fluid, semen, milk, cell medium, tissue extract, and cancer tissue.

4. The light-controllable self-assembled complex of claim 1, wherein the self-assembled complex further comprises a delivery substance therein, wherein the delivery substance is released upon swelling of the self-assembled complex, and remains inside the self-assembled complex when the self-assembled complex is in a deswollen state.

5. The light-controllable self-assembled complex of claim 4, wherein the delivery substance comprises at least one of a protein, a nucleic acid, and a small molecule drug, wherein the protein comprises at least one of cytokines, chemokines, antibodies for anticancer immunotherapy, growth factors, botulinum toxins, and antigens, and the nucleic acid comprises at least one of messenger RNA (mRNA), micro RNA, and plasmid DNA.

6. The light-controllable self-assembled complex of claim 1, wherein the photoswitchable isomer is converted to the cis-isomer by ultraviolet light to swell the self-assembled complex, and is converted to the trans-isomer by visible light to deswell the self-assembled complex.

7. The light-controllable self-assembled complex of claim 1, wherein the polymer comprises at least one of carboxymethyl cellulose, hyaluronic acid, alginate, poly(L-glutamic acid), poly(L-aspartic acid), polyacrylic acid (PAA), gelatin, and collagen.

8. The light-controllable self-assembled complex of claim 1, wherein the ligand comprises at least one of brassicasterol, stigmasterol, ascorbic acid, cyanocobalamin, tocopherol, retinol, aminobutyric acid, amine-Boc hydrochloride, cyclic RGD, VH 032 amide-PEG2-amine, histamine, tyramine, β-phenylethylamine, tryptamine, serotonin, putrescine, cadaverine, spermidine, and spermine.

9. The light-controllable self-assembled complex of claim 1, wherein the trans-isomer of the photoswitchable isomer has a structure represented by the following Formula 1: 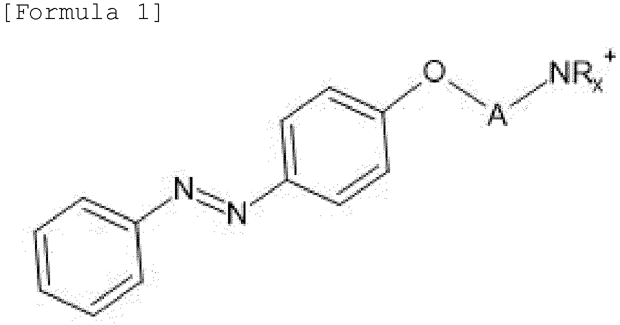 wherein A is any one of C0 to C15, R is any one of hydrogen, methyl, ethyl, and propyl, and X is an integer ranging from 1 to 3.

10. The light-controllable self-assembled complex of claim 1, wherein
the photoswitchable isomer is converted to the cis-isomer to swell the self-assembled complex and promote macrophage adhesion to the self-assembled complex and macrophage M2 polarization, or
the photoswitchable isomer is converted to the trans-isomer to deswell the self-assembled complex, inhibit macrophage adhesion to the self-assembled complex and promote macrophage M1 polarization.

11. A light-controllable nanosystem comprising:
the light-controllable self-assembled complex according to any one of claims 1 to 10;
a substrate on which at least a portion of the self-assembled complex is provided; and
upconversion nanoparticles bound to the substrate,
wherein the upconversion nanoparticle comprises a core-shell portion and a coating portion with which the surface of the core-shell portion is coated,
wherein the core-shell portion is configured to absorb infrared light and upconvert the absorbed infrared light into at least one of ultraviolet light and visible light,
the coating portion is configured to absorb the visible light upconverted by the core-shell portion, and
the upconversion nanoparticle is configured to emit the ultraviolet light upconverted by the core-shell portion.

12. The light-controllable nanosystem of claim 11, wherein the core-shell portion comprises a core provided in a central portion thereof, and a shell provided to cover an outer surface of the core,
wherein the core comprises at least one of NaYbF₄, NaYF₃, NaGdF₄, KGdF₄, YOF, BaLaF₅, LaF₃, NaLuF₄ and SrF₂,
the shell comprises at least one of CaF₂, Na(Yb,Gd)F₄, NaGdF₄ and TiO₂,
the core-shell portion is doped with a dopant, and
the dopant comprises at least one of Yb³⁺, Tm³⁺, Ln³⁺ and Er³⁺.

13. The light-controllable nanosystem of claim 11, wherein the coating portion comprises at least one of a first coating agent and a second coating agent,
wherein the first coating agent comprises at least one of β-carotene, curcumin, fluorescent protein, R-phycoerythrin, rhodamine, FDB-003, FDB-004, and FDB-005, and
the second coating agent comprises at least one of Tween 20, Tween 80, sodium oleate, sodium dodecyl sulphate (SDS), sodium dodecyl benzene sulphonate, sodium stearate, dodecylamine hydrochloride, span-60, span-80, polyethylene oxide, and dodecyl bataine.

14. The light-controllable nanosystem of claim 11, wherein the nanosystem works in a living body and is controlled by light applied from outside the living body,
wherein, when infrared light is applied to the nanosystem in the living body from outside the living body, the photoswitchable isomer in the living body is converted to the cis-isomer to swell the self-assembled complex in the living body, and
when visible light is applied to the nanosystem in the living body from outside the living body, the photoswitchable isomer in the living body is converted to the trans-isomer to deswell the self-assembled complex.

15. The light-controllable nanosystem of claim 11, wherein
the self-assembled complex reversibly swells and deswells in response to light in an environment with a aqueous liquid,
wherein, when the nanosystem is irradiated with infrared light, the self-assembled complex swells and absorbs the aqueous liquid, and
when the nanosystem is irradiated with visible light, the self-assembled complex deswells and releases the aqueous liquid.
